(19) Europäisches Patentamt
European Patent Office
Office européen des brevets

(11) **EP 2 649 041 B1**

(12) **EUROPÄISCHE PATENTSCHRIFT**

(45) Veröffentlichungstag und Bekanntmachung des Hinweises auf die Patenterteilung:
**08.06.2016 Patentblatt 2016/23**

(51) Int Cl.:
*C07C 209/36* [(2006.01)]    *C07C 211/50* [(2006.01)]

(21) Anmeldenummer: **11790975.4**

(22) Anmeldetag: **05.12.2011**

(86) Internationale Anmeldenummer:
**PCT/EP2011/071707**

(87) Internationale Veröffentlichungsnummer:
**WO 2012/076449 (14.06.2012 Gazette 2012/24)**

(54) **VERFAHREN ZUR HERSTELLUNG VON AROMATISCHEN AMINEN**

METHOD FOR PRODUCING AROMATIC AMINES

PROCÉDÉ DE PRODUCTION D'AMINES AROMATIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.12.2010 EP 10193860**

(43) Veröffentlichungstag der Anmeldung:
**16.10.2013 Patentblatt 2013/42**

(73) Patentinhaber: **BASF SE**
**67056 Ludwigshafen am Rhein (DE)**

(72) Erfinder:
• **HAASE, Stefanie**
**01900 Bretnig - Hauswalde (DE)**
• **KADIJEVIC, Dusko**
**68305 Mannheim (DE)**
• **MERTEN, Anne-Kathrin**
**01979 Lauchhammer (DE)**
• **ZOELLINGER, Michael**
**73054 Eislingen (DE)**
• **RAICHLE, Andreas**
**67063 Ludwigshafen (DE)**
• **SCHOCKER, Alexander**
**CH-8105 Watt (CH)**
• **MÜHLBEYER, Sandra**
**67227 Frankenthal (DE)**
• **WIEDENHOFF, Olaf**
**68789 St. Leon-Rot (DE)**
• **COELHO TSOU, Joana**
**B-1190 Brüssel (BE)**

(56) Entgegenhaltungen:
WO-A1-03/066571          WO-A1-2006/089906
WO-A1-2011/144481     US-A- 5 856 577

**Beschreibung**

Die Erfindung betrifft ein Verfahren zur Herstellung von aromatischen Aminen durch Hydrierung von Nitroaromaten in Gegenwart von Katalysatoren.

Die Herstellung von Aminen, insbesondere von aromatischen Mono- und/oder Polyaminen durch katalytische Hydrierung von Mono- und/oder Polynitroverbindungen ist aus dem Stand der Technik bekannt.

DE-OS 2 044 657 beschreibt ein Verfahren zur Herstellung von Toluylendiamin (TDA) durch Hydrieren von Dinitrotoluol (DNT) in Gegenwart von Nickel oder Ruthenium enthaltenden Hydrierkatalysatoren.

EP-B1 1 138 665 beschreibt ein Verfahren zur katalytischen Hydrierung von aromatischen Nitroverbindungen, bei dem die Hydrierung kontinuierlich mit einem Katalysator realisiert wird, der mindestens Nickel und gegebenenfalls Aluminium umfasst. Nach der Durchführung der Hydrierung wird der Katalysator in einer Trennzone von der Reaktionsmischung getrennt.

US 5 856 577 A beschreibt ein Verfahren zur Herstellung von Fluoroanilinen aus Fluoronitrobenzolen.

Das bei der Durchführung der Hydrierung von Nitroaromaten in einem Reaktor erhaltene Reaktionsgemisch enthält außer den aromatischen Aminen auch Nitro- und Nitrosoverbindungen, die zum Beispiel die als Edukte eingesetzten Nitroaromaten oder in dem Reaktor gebildete Zwischenprodukte umfassen. Nitro- und Nitrosoverbindungen können sich insbesondere beim Erhitzen explosionsartig zersetzen. Aus Sicherheitsgründen ist daher eine Überwachung des Reaktionsgemisches in Bezug auf die darin vorhandene Konzentration an Nitro- und Nitrosoverbindungen wichtig. Das Sicherheitsrisiko steigt in dem Maße an, in dem die Reaktorgröße zunimmt und die Verweilzeiten in dem Reaktor reduziert werden. Es muss sichergestellt werden, dass diese explosiven Verbindungen vollständig im Reaktor umgesetzt werden, bevor das Reaktionsgemisch zum Beispiel einer nachfolgenden Destillation zugeführt wird.

Des Weiteren ergibt sich bei der katalytischen Hydrierung von Nitroaromaten mit Nickel enthaltenden Katalysatoren das Problem, dass die Katalysatoren mit der Zeit desaktiviert werden, wobei Nitroaromaten die Katalysatordesaktivierung beschleunigen. Je geringer die Aktivität der Katalysatoren ist, umso geringer ist der zu Aminen umgesetzte Anteil der Edukte, so dass der in dem Reaktor verbleibende Anteil an nicht umgesetzten Nitroaromaten steigt, was wiederum die Desaktivierung beschleunigt. Daher ist eine Überwachung der Katalysatoraktivität notwendig, insbesondere um dem Reaktor eine ausreichende Menge an unverbrauchtem Katalysator zuzuführen.

Bisher wurde die Überwachung der Konzentration an Nitroaromaten entweder mit UV/VIS-Methoden nach der Abtrennung des Katalysators direkt in dem Reaktionsgemisch durchgeführt, wobei zu beachten ist, dass die dabei erreichten Nachweisgrenzen deutlich über der Nitroaromatengrenzkonzentration für die Katalysatordesaktivierung liegt, oder durch manuelle Probennahme und Aufbereitung und Bestimmung im Labor durch polarographische oder chromatographische Methoden. Nachteilig an der manuellen Probennahme sind der hohe personelle Einsatz und die Gefährdungen, die aus der Probennahme folgen. Zur Steuerung des Prozesses sind diese manuellen Verfahren aufgrund der zeitlichen Verzögerung der Bereitstellung von Analysenergebnissen von großem Nachteil.

Bei solchen manuellen Steuerungen kann es daher während relativ langer Zeiträume dazu kommen, dass das hergestellte aromatische Amin/Wasser-Gemisch erhöhte Konzentrationen an Nitroaromaten enthält und diese Nitroaromaten eine merkliche irreversible Erniedrigung der Katalysatoraktivität mitbringt. Dies kann bis zum völligen Erliegen der katalytischen Aktivität und somit zum Ausfall der Produktion führen.

Diese aromatischen Amine können z. B. Naphthyldiamine, Xylylendiamine, Toluylendiamine, Aniline und Toluidine sowie weitere aromatische Amine und Mischungen sein. Die entsprechenden Nitroverbindungen sind Dinitronaphthaline, Dinitroxylole, Dinitrotoluole (DNT), Aminonitrotoluole (ANT), Mononitrobenzole und Mononitrotoluol.

Die Herausforderung an eine Online-Bestimmung von z. B. Dinitrotoluol (DNT) in der Matrix Toluylendiamin (TDA)/Wasser mit metallischem Katalysator sind sehr hoch aufgrund des hohen Schmelzpunktes der Probe von ca. 80 °C und der Zusammensetzung der Probe aus vielen Isomeren, dem Feststoffgehalt durch metallischen Katalysator von ca. 0,1 bis 15 Gew.-% und der Anforderung, DNT im Spurenbereich von bevorzugt 3 bis 30 ppm zu messen.

Gemäß Stand der Technik wird die Aktivität des Katalysators anhand von Gaschromatographie (GC)-Proben verfolgt, zum Beispiel in dem Verfahren gemäß Beispiel 1 der WO 03/066571 A1.

In der WO 2006/089906 ist die Bestimmung der Konzentration der Nitro- und Nitrosoverbindungen mithilfe der UV/VIS-Spektroskopie beschrieben. Hierzu ist eine Abtrennung des Katalysators vom Reaktionsgemisch erforderlich. Die Nachweisgrenze der Bestimmung beträgt typischerweise 40 bis 50 ppm.

Die bekannten Verfahren sind durch aufwändige Probenzuführung und -konditionierung, Störanfälligkeit und größeren Verbrauch an Hilfsstoffen und Verbrauchsrnaterialien gekennzeichnet.

Die kontinuierliche und schnelle Überwachung und Regelung der Ist-Konzentration an Nitroaromaten im Hydrierprozess ist insbesondere für die Herstellung von aromatischen Aminen von Bedeutung.

Oft werden die hergestellten Amine und Diamine zu Isocyanaten weiterverarbeitet.

Aufgabe der vorliegenden Erfindung ist es, ein Verfahren zur Herstellung von aromatischen Aminen durch Hydrierung von Nitroaromaten in Gegenwart von Katalysatoren in einem Reaktor bereitzustellen, das eine einfache Online-Überwachung der Konzentration von Nitro- und Nitrosoverbindungen in einem in dem Reaktor enthaltenen Reaktionsgemisch erlaubt.

Die Nachweisgrenze soll gegenüber der UV/VIS-Spektroskopie gesenkt und damit die Empfindlichkeit erhöht sein.

Die Aufgabe besteht ferner darin, eine quasikontinuierliche oder Online-Überwachung der Katalysatoraktivität in dem Reaktor zu ermöglichen, die eine Abnahme der Katalysatoraktivität bereits detektiert, bevor die Nitroaromaten-Konzentration einen Wert erreicht, bei dem sie die Katalysatordesaktivierung weiter beschleunigt und damit eine Negativspirale in Gang setzt. Im Falle von DNT bedeutet dies z. B., dass dieses bereits in einem Konzentrationsbereich unter 30 ppm und bevorzugt zwischen 3 ppm und 30 ppm bestimmt werden muss. Eine weitere Aufgabe besteht darin, die manuelle Probennahme und die Offline-Analytik von Nitroaromaten zu ersetzten, da eine manuelle Probennahme ein erhebliches Sicherheitsrisiko darstellt und durch eine Automatisierung der Messung sehr viel Laboranalytik eingespart werden kann und gleichzeitig die Informationen schneller für eine Prozessführung bereitgestellt werden können.

Die Aufgabe wird erfindungsgemäß gelöst durch ein Verfahren zur Herstellung von aromatischen Aminen durch Hydrierung von Nitroaromaten in Gegenwart von Katalysatoren, wobei in einem Reaktor ein fluides, Amine enthaltendes Reaktionsgemisch entsteht, bei dem ein Gaschromatogramm des Reaktionsgemisches zur Konzentrationsbestimmung von Nitro- und Nitrosoverbindungen in dem Reaktionsgemisch erstellt oder ausgewertet wird, wobei das Ergebnis der Konzentrationsbestimmung der Nitro- und Nitrosoverbindungen zur vorzugsweise automatisierten, Prozess-Steuerung oder Regelung der Hydrierung verwendet wird, dadurch gekennzeichnet, dass Proben automatisiert, über einen Bypass dem Gaschromatographen online zugeführt werden.

Die Prozess-Steuerung oder Regelung der Hydrierung beinhaltet vorzugsweise eine Änderung der Zudosierung von Katalysator und/oder Nitroaromaten in den Reaktor.

Eine Flächenbestimmung der entsprechenden Peaks im Chromatogramm kann zur Konzentrationsbestimmung einzelner Komponenten des Reaktionsgemisches herangezogen werden.

Die Probe wird über einen Bypass automatisiert dem Chromatographen online zugeführt, so dass sich sehr zeitnah ein genaues Abbild der Konzentrationsverhältnisse im Reaktor ergibt.

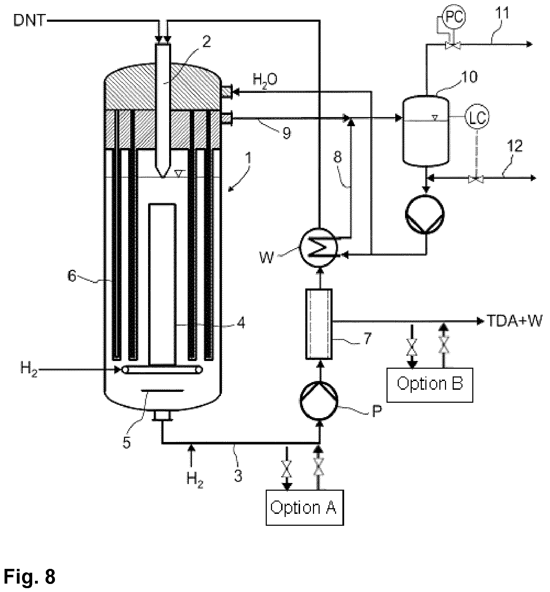

Die Probenahme für die chromatographische Analyse "online" bedeutet, dass die Probennahme in einem Bypass des flüssigen Produktaustrags zwischen Reaktor (z. B. Rührkessel, Schlaufenreaktor usw.) und Produktabtrenneinheit (z. B. Membranfilter, Schwerkraftabscheider usw.), vorzugsweise mit einem zusätzlichen (Membran)filter, erfolgt (Option A) oder in einem Bypass des flüssigen Produktaustrags nach der Produktabtrenneinheit (z. B. Membranfilter) erfolgt (Option B) (siehe Fig. 8).

Option A kann vorzugsweise mit einem Partikelfilter und einem nachfolgenden automatisierten Flüssigdosierventil zur Probennahme ausgerüstet sein. Durch die Probenahme im flüssigen Produktaustrag zwischen Reaktor und Produktabtrenneinheit erfolgt die Messung bevorzugt an einem Ort geringer DNT-Konzentration, also typischerweise an einer der DNT-Zudosierung abgewandten Stelle über eine Katalysatorabtrenneinheit. Durch die Probenahme im flüssigen Produktaustrag erfolgt gleichsam eine abschließende Kontrolle der Misch- und Reaktionsprozesse in den vorgelagerten Reaktoren. Messungen der DNT-Konzentration an verschiedenen Stellen direkt im Reaktor sind technisch sehr viel aufwändiger. Die einzustellenden Konzentrationsbereiche würden sich mit zunehmender Nähe zum Ort der DNT-Zudosierung (und/oder zu schlechter durchströmten Bereichen des Reaktors) erheblich erhöhen. Nachteilig an dieser Ausführungsform ist die Verwendung einer zusätzlichen Abtrenneinheit für den Katalysator. Bevorzugte Ausführungsformen der zusätzlichen Katalysatorabtrenneinheit sind keramische Membranfilter.

Bei Option B erfolgt die Probenahme für die chromatographische Analyse nach der Produktabtrenneinheit. Somit ist für die Messung keine zusätzliche Katalysatorabtrenneinheit erforderlich. Nachteilig dabei ist die Verschlechterung der Reaktionszeit für die Regelung der Hydrierung, da die Probe an einer weiter vom Reaktor entfernten Stelle genommen wird. Vorzugsweise erfolgt die Dosierung des Reaktionsgemisches in den Gaschromatographen mittels eines auf mindestens 80 °C beheizten automatischen Flüssigdosierventils.

Die Probe des Reaktionsgemisches kann bei der Probennahme bis zur Analyse und gegebenenfalls Rückführung in das Reaktionsgemisch bei einer Temperatur von mindestens 80 °C gehalten werden oder nach der Probennahme mit einem Lösungsmittel verdünnt werden. Diese Verdünnung kommt insbesondere bei HPLC-Verfahren zur Anwendung.

Die Aufgabe wird zudem gelöst durch ein Verfahren zur Regelung der Konzentration von Nitro- und Nitrosoverbindungen nach dem erfindungsgemäßen Verfahren zur Herstellung von aromatischen Aminen durch Hydrierung von Nitroaromaten in Gegenwart von Katalysatoren, wobei in einem Reaktor ein fluides, Amine enthaltendes Reaktionsgemisch entsteht, umfassend die folgenden Schritte:

a) Aufnahme eines Gaschromatogramms des Reaktionsgemisches, wobei die Proben automatisiert über einen Bypass dem Gaschromatographen alleine zugeführt werden,

b) Eingabe des in Schritt a) aufgenommenen Gaschromatogramms in eine Gaschromatogramm-Auswerteeinheit (Auswertesoftware) und/oder in ein chemometrisches Kalibrationsmodell zur Bestimmung der Ist-Konzentration der Nitro- und Nitrosoverbindungen im Reaktionsgemisch,

c) Bestimmung einer Soll-Ist-Abweichung der Konzentration der Nitro- und Nitrosoverbindungen im Reaktionsgemisch,

d) Regelung eines oder mehrerer Prozessparameter der Hydrierung, basierend auf der in Schritt c) bestimmten Soll-Ist-Abweichung.

In Schritt b) kann zur Bestimmung der Ist-Konzentration der Nitro- und Nitrosoverbindungen im Reaktionsgemisch das aufgenommene Gaschromatogramm in ein chemometrisches Kalibrationsmodell eingegeben werden. Dabei erfolgt eine simultane Mehrkomponentenanalytik, um eine Quantifizierung der verschiedenen Reaktionsspezies (z. B. Edukte wie DNT und Zwischenprodukte wie ANT) im Prozess online durchzuführen. Um die Quantifizierung durchführen zu können, bedarf es einer vorherigen chemometrischen Kalibrierung der Analysenmethode. Einen Überblick über die Verwendung von multivariaten chemometrischen Kalibrationsmodellen in der analytischen Chemie gibt "Multivariate Kalibration", Jörg-Peter Conzen, 2001, ISBN 3-929431-13-0. In Schritt d) wird vorzugsweise die Katalysatorzudosierung und/oder die Zudosierung von Nitroaromaten verändert bzw. geregelt. Häufig wird bei einer gegenüber dem Sollwert erhöhten Nitroaromaten-Konzentration die Katalysatorzudosierung erhöht und/oder die Zudosierung von Nitroaromaten vermindert.

Die Aufgabe wird ferner gelöst durch eine Vorrichtung zur Durchführung des vorstehenden Verfahrens zur Herstellung von aromatischen Aminen mit einem Reaktor zur Hydrierung von Nitroaromaten in Gegenwart von Katalysatoren, wobei ein Reaktionsgemisch entsteht, mit einem Gaschromatographen zur Erstellung eines Gaschromatogramms zur Konzentrationsbestimmung von Nitro- und Nitrosoverbindungen in dem Reaktionsgemisch, wobei die Vorrichtung eingerichtet ist, um die Aufnahme von Gaschromatogrammen von Proben, die automatisiert über einen Bypass dem Gaschromatographen online zugeführt werden, vorzunehmen, mit einer Auswerteeinheit zur Konzentrationsbestimmung von Nitroaromaten, sowie einer Regelungseinheit zur Regelung der Katalysatorzudosierung und/oder Zudosierung von Nitroaromaten.

Die Vorrichtung umfasst vorzugsweise:

a) Mittel (202, 204) zur Aufnahme eines Gaschromatogramms des Reaktionsgemisches von Proben, die automatisiert über einen Bypass dem Gaschromatographen online zugeführt werden,

b) eine Gaschromatogramm-Auswerteeinheit (Auswertesoftware) (206) und/oder ein chemometrisches Kalibrationsmodell (210) zur Auswertung des Gaschromatogramms zur Bestimmung einer Ist-Konzentration an Nitro- und Nitrosoverbindungen im Reaktionsgemisch aus dem Chromatogramm,

c) Mittel (212) zur Bestimmung einer Soll-Ist-Abweichung,

d) Mittel (212) zur Regelung eines oder mehrerer Prozessparameter der Hydrierung, insbesondere der Dosierung der aromatischen Nitroverbindung (214), und/oder Dosierung des Katalysators (216) basierend auf der Soll-Ist-Abweichung.

Unter Nitroverbindungen werden in diesem Zusammenhang vorzugsweise aromatische Verbindungen verstanden, in denen ein Wasserstoffatom durch eine Nitrogruppe (NO$_2$-Gruppe) ersetzt ist. Unter Nitrosoverbindungen sind organische Verbindungen zu verstehen, die die Nitrosogruppe (NO-Gruppe) an ein aromatisches Kohlenstoffatom gebunden enthalten. Unter Aminen sind Mono-, Di- und Polyamine zu verstehen. Das fluide Reaktionsgemisch kann sowohl flüssig als auch gasförmig sein.

Erfindungsgemäß wird die Konzentrationsbestimmung der Nitro- und Nitrosoverbindungen durch quantitative Auswertung eines Gaschromatogramms des Reaktionsgemisches durchgeführt, in der Regel durch Integration der Peakflächen zu den einzelnen zu analysierenden Verbindungen.

Die Automatisierbarkeit der Abläufe in einer chromatographischen Analyse und die durch Anwendung moderner chromatographischer Techniken erzielbaren kurzen Analysenzeiten gestatten es, einen kontinuierlichen Betrieb des Chromatographen (online im Bypass) zu realisieren. Die Ergebnisse werden zeitdiskret zur Verfügung gestellt. Der Einsatz eines vollautomatisch arbeitenden Chromatographen führt zu deutlichen Kostenvorteilen gegenüber personalintensiven Laboranalysen und liefert durch die quasikontinuierliche Bereitstellung von Analysenwerten ein sehr detailliertes Abbild des betrachteten Prozesses.

Eine chromatographische Prozessanalysenanlage besteht aus einer dem Anwendungsfall angepassten Einrichtung zur Probenahme, -aufbereitung und gegebenenfalls zur Umschaltung verschiedener Probenströme, dem eigentlichen Analysator und einer Einrichtung zur Messdatenverarbeitung und -ausgabe.

Bei der Modifikation von Laborgaschromatographen für die Online-Analytik sind die spezifischen Anforderungen an die Probenzufuhr und -aufbereitung, die Injektion sowie die Datenauswertung und -verwaltung zu berücksichtigen.

Dazu wird eine graphische Benutzeroberfläche "GC-Manager" verwendet (GC-Manager ist eine eingetragene Marke der BASF SE beim Deutschen Patent- und Markenamt, München). Der "GC-Manager" steuert weitere (Hersteller-)Programme fern und stellt damit die volle Funktionalität des Gerätes und den problemlosen Methodentransfer sicher. Die Datenerfassung und -verarbeitung wird vom "GC-Manager" kontrolliert. Die Ergebnisse einer GC-Analyse lassen sich in Datenbanken oder benutzerdefinierte MS Excel®Tabellen automatisch übertragen. Darüber hinaus visualisiert der "GC-Manager" auch weitere wichtige Informationen, wie z. B. die Ventilstellungen, den ausgewählten Probenstrom oder den Status der Analyse. Eine weitere Besonderheit der Software ist die Kommunikation mit Prozessleitsystemen über analoge oder digitale Schnittstellen. Eine direkte Übertragung der Konzentrationswerte in das Prozessleitsystem und folglich eine Regelung des Prozesses sind damit möglich.

Für die Analytik eignet sich sowohl die Hochleistungsflüssigkeitschromatographie (HPLC) sowie davon abgeleitete Technologien wie UPLC, UHPLC (Ultrahochleistungsflüssigkeitschromatographie) als auch die Gaschromatographie. Entsprechende Instrumente für den Laboreinsatz als auch für Prozessmessungen sind kommerziell erhältlich (z. B. von Agilent Technologies (Laborgeräte) oder Siemens AG (Prozessgeräte)). Der Vorteil einer chromatographischen Analytik liegt dabei vor allem auch darin, dass die verschiedenen auftretenden Isomere des Edukts/der Edukte, der partiell hydrierten Reaktionsprodukte oder der Isomere der vollständig hydrierten Reaktionsprodukte detektiert, quantifiziert und mittels einer nachgeschalteten massenspektrometrischen Detektion ggf. auch identifiziert werden können. Bestimmte Isomere können zu ungewünschten Nebenreaktionen führen, die eine stärkere Schädigung des Katalysators zur Folge haben als andere. Durch die chromatographische Analytik wird ersichtlich, ob die verschiedenen Isomere der Edukte oder Zwischenprodukte unterschiedlich abreagieren.

Nach einer bevorzugten Ausführungsform der Erfindung wird mittels eines Gaschromatographen (GC) der Gehalt an Nitroaromaten und Nitrosoaromaten während der Hydrierung online im Hydrierbad bestimmt. Dabei wird das zu analysierende Gemisch mit einem Schmelzpunkt von etwa 80 °C in flüssiger Form dem Instrument, vorzugsweise automatisiert über ein Flüssigdosierventil, zugeführt und dann flüssig injiziert. Die Zufuhr der Probe in flüssiger Form kann auf zwei unterschiedlichen Wegen geschehen:

1. Verdünnung des Reaktionsgemisches mit einem Lösungsmittel (beispielsweise Anilin im Verhältnis 1 : 4 bis 1 : 1) und Zufuhr bei z. B. Raumtemperatur

2. kontinuierliche, durchgehende Beheizung der Zu- und Ableitungen des Reaktionsgemisches auf mindestens 80 °C, so dass das Gemisch flüssig bleibt, wodurch die automatisierte Probennahme vereinfacht wird.

Ein Erstarren des Reaktionsgemisches unterbricht die kontinuierliche Zufuhr zum Chromatographen (Analysengerät), zieht eine aufwändige Reinigung und Erneuerung der Leitung nach sich und unterbricht die Online-Analytik. In der bevorzugten Ausführungsform erfolgt die Zufuhr und Dosierung nach der Option 2, bei der gegenüber der Option 1 auf eine hohe Menge an Verbrauchsmaterialien (z. B. Lösungsmittel Anilin) verzichtet werden kann.

In der bevorzugten Umsetzung erfolgt die Dosierung/Injektion nach der durch Option 1 oder Option 2 konditionierten Probe in den Gaschromatographen mittels eines automatisierten und beheizten Flüssigdosierventils (Siemens AG). Ein kleines Volumen der Probe (ca. 0,6 $\mu$l) wird verdampft und gasförmig auf die Trennsäule gegeben.

Für die instrumentelle Umsetzung bieten sich Labor- oder Prozessinstrumente an. Vorteilhaft am Einsatz eines Laborgeräts ist die schnelle Übertragbarkeit der vorhandenen Methode auf eine Online-Applikation im Prozess. Bei einem Laborinstrument kann zudem durch eine programmierte Temperaturerhöhung im Säulenofen die Trenngeschwindigkeit gegenüber einem Prozessinstrument erhöht werden.

Vom GC werden die Ergebnisse der Analytik (Gehalt an Nitroaromaten und Nitrosoaromaten, aus den entsprechenden Peakflächen im Chromatogramm bestimmt) über typische Schnittstellen der Prozessleittechnik an das Prozessleitsystem übertragen.

Es ist im erfindungsgemäßen Verfahren möglich, die Messungen ohne vorherige Abtrennung des Katalysators durchzuführen. Aufgrund des Feststoffanteils - insbesondere kleinerer Katalysatorpartikel (Größenordnung < ca. 25 $\mu$m) - hat sich gezeigt, dass die Messungen mit vorheriger Abtrennung des Katalysators vorteilhafter durchgeführt werden können. Oft wird in einem Reaktor mit externer Schlaufe das Umsetzungsprodukt über einen Querstromfilter ausgetragen. Vorteilhaft wird der Bypass hinter dem Querstromfilter angeordnet. Hierdurch ist es möglich, dass die gaschromatographische Analytik ohne weitere wesentliche Probenvorbereitung durchgeführt wird. Hierzu wird ein Strom des Reaktionsgemisches abgezweigt und zum Gaschromatographen geführt. Vorzugsweise passiert dies online, d. h. in einem Bypass. Es hat sich dabei als besonders vorteilhaft gezeigt, ein automatisiertes Flüssigdosierventil in einem Bypass zu installieren.

Die Konzentration der Feststoffteilchen sollte vorzugsweise 15Gew.-% nicht überschreiten. Entsprechend hat es sich als besonders vorteilhaft gezeigt, die GC-Messung in einen Bypass zu installieren und wenn immer möglich mit geeigneten Partikelfiltern auszustatten. Auf diese Weise kann der Großteil des enthaltenen Katalysators vor Durchführung der Messung abgetrennt, Störungen der Messungen sicher vermieden und der Reinigungs- und Wartungsaufwand stark reduziert werden.

Das Abtrennverfahren für den Katalysator kann aus der Gruppe Membranfiltration, Sedimentation und Zentrifugation ausgewählt sein oder mit einem beliebigen anderen, im Stand der Technik bekannten Verfahren. Das Abtrennen eines in einem Reaktionsgemisch enthaltenen Katalysators ist zum Beispiel aus DE-A1 32 45 318, DE-A1 30 40 631 oder WO 03/066571 bekannt. Es ist auch eine Kombination von Abtrennverfahren möglich (zum Beispiel Sedimentation und anschließende Membranfiltration), um eine möglichst vollständige Abtrennung des Katalysators aus dem Reaktionsgemisch zu erreichen, bevor eine chromatographische Analytik durchgeführt wird. Nach einer bevorzugten Ausführungsform der Erfindung wird die Konzentration über die Flächenauswertung des aufgenommenen Gaschromatogramms bestimmt. Die stoffliche Matrix ist durch den Hydrierprozess vorgegeben und von den Prozessparametern abhängig. Die gemessenen Werte werden zu den Ergebnissen aus Laboruntersuchungen (Kalibriergeraden) referenziert.

Dies erfolgt in der Weise, dass im Labor mit Hilfe von künstlich hergestellten Proben mit bekanntem Gehalt an Nitroaromaten und Nitrosoaromaten GC-Messungen durchgeführt werden und Kalibriergeraden erstellt werden.

Mit Hilfe dieses Verfahrens lassen sich bereits kleine Mengen von Nitroaromaten wie Dinitrotoluol im Konzentrationsbereich von 3 - 30 ppm im Produkt von Hydrierprozessen gaschromatographisch in der stofflichen Matrix bestimmen.

Zur Konzentrationsbestimmung der Nitro- und Nitrosoverbindungen kommen übliche Gaschromatographen und Trennsäulen zum Einsatz, z. B. von Agilent Technologies (Labor-GC), von Restek (Säulen), von Siemens AG (Prozessgeräte).

Eine Vorrichtung zur IR- und/oder VIS-Messung umfasst vorzugsweise:

a) Mittel (202, 204, 206, 208) zur Aufnahme eines Infrarot- oder VIS-Spektrums des Reaktionsgemisches,

b) ein chemometrisches Kalibrationsmodell (210) zur Bestimmung einer Ist-Konzentration an Nitro- und Nitrosoverbindungen im Reaktionsgemisch aus dem Infrarot-Spektrum,

c) Mittel (212) zur Bestimmung einer Soll-Ist-Abweichung,

d) Mittel (212) zur Regelung eines oder mehrerer Prozessparameter der Hydrierung, insbesondere der Dosierung der aromatischen Nitroverbindung (214), basierend auf der Soll-Ist-Abweichung.

Die Konzentrationsbestimmung der Nitro- und Nitrosoverbindungen kann durch Messung der Absorption von Infrarot-Strahlung im Reaktionsgemisch durchgeführt werden. Es kann sich um Nah-Infrarot (NIR)-Strahlung, Mittel-Infrarot-Strahlung oder Raman-Strahlung handeln. Bevorzugt wird mit NIR-Strahlung gearbeitet. Diese Messung kann durch VIS-Messungen ergänzt oder durch diese ersetzt werden.

Aus einer gemessenen Absorption von IR-Strahlung durch das Reaktionsgemisch lässt sich die Konzentration von Nitro- und Nitrosoverbindungen in dem Reaktionsgemisch bestimmen. Wenn monochromatische IR-Strahlung mit einer Anfangsintensität $I_0$ eine verdünnte Lösung eines absorbierenden Stoffes (zum Beispiel das Reaktionsgemisch) der Dicke d durchdringt, so beschreibt das Lambert-Beer'sche Gesetz die Absorption der Strahlung durch die Lösung. Das Lambert-Beer'sche Gesetz lautet:

$$2,3 \bullet \log \frac{I_0}{I} = E = \varepsilon \bullet d \bullet c$$

wobei

$I_0$: Intensität der Strahlung vor Eintritt in die Lösung
$I$: Intensität der Strahlung nach Durchdringen der Schichtdicke d
$d$: Schichtdicke (zum Beispiel Küvettenmaß) des Fluides (z.B. der Lösung) in cm
$c$: Konzentration der absorbierenden Substanz in mol/l
$\varepsilon$: molarer Extinktionskoeffizient in l/(mol x cm) (Stoffkonstante)
$E$: Extinktion.

Es ergibt sich ein linearer Zusammenhang zwischen der Konzentration des Fluides und der Extinktion. Dadurch ist die Bestimmung der Konzentration einer absorbierenden Probe durch die Messung der Extinktion (zum Beispiel mit einem Spektralphotometer) mittels einer Eichkurve oder bekannten Extinktionskoeffizienten möglich (Photometrie). Die Abtrennung der Katalysatoren aus dem Reaktionsgemisch vor der Absorptionsbestimmung kann die Aufarbeitung des Endprodukts vereinfachen. Anders als bei der UV/VIS-Spektroskopie muss das Reaktionsgemisch zur Absorptionsmessung nicht weitgehend von den schwarzen Katalysatorsteilchen befreit sein, da diese die IR-Spektroskopie nicht wesentlich stören.

Damit ist es möglich, die Messungen ohne vorherige Abtrennung des Katalysators durchzuführen. Dies erniedrigt die Anforderungen an die probenvorbereitende Messtechnik erheblich. Hierdurch ist es möglich, dass die Messung der Absorption von Infrarot-Strahlung ohne Probennahme inline durchgeführt wird. Hierzu wird ein Strom des Reaktionsgemisches abgezweigt und an einer Messzelle zur Messung der Infrarot-Strahlung vorbeigeführt. Vorzugsweise passiert dies online in einem Bypass. Es hat sich dabei als besonders vorteilhaft gezeigt, die Infrarot-Messsonde in einem Bypass zu installieren, der mit, vorzugsweise automatischen, Ventilen vor und hinter der Messsonde ausgerüstet ist. Zur Messung können die Ventile geschlossen werden, so dass sich der im Reaktionsgemisch enthaltene Katalysator absetzen kann. Hierdurch werden noch störungsfreiere Messungen der IR-Absorption möglich.

Zur Konzentrationsbestimmung der Nitro- und Nitrosoverbindungen kann ein Absorptionsspektrum in einem Wellenlängenbereich der IR-Strahlung oder die Absorption (Extinktion) von IR-Strahlung mit einer Wellenlänge gemessen werden. Vorzugsweise erfolgt die Messung monochromatisch (mit Strahlung einer ausgewählten Wellenlänge). Dies ist ausreichend, um die Konzentration der Nitro- und Nitrosoverbindungen in dem Reaktionsgemisch zu bestimmen. Die Wellenlänge der Strahlung wird so ausgewählt, dass Beiträge anderer Komponenten des Reaktionsgemisches, insbesondere der Amine zu der Absorption der Strahlung möglichst gering sind.

Bei der Messung eines IR-Spektrums erfolgt die Messung vorzugsweise in einem Wellenlängenbereich von 600 bis 1365 nm, besonders bevorzugt von 650 bis 1200 nm. Auch eine monochromatische Messung liegt vorzugsweise bei einer Wellenlänge in diesem Wellenlängenbereich.

Die Messung des Absorptionsspektrums in einem Wellenlängenbereich der IR-Strahlung hat den Vorteil, dass fehlerhafte Messungen, die zum Beispiel durch Gasblasen in dem Reaktionsgemisch verursacht werden können, leicht erkannt und nicht zur Konzentrationsbestimmung herangezogen werden. Eine solche Messung eines Absorptionsspektrums erfordert jedoch ein aufwendigeres Photometer, bei dem sowohl die darin enthaltene Lichtquelle, als auch der Detektor einen solchen Wellenlängenbereich abdecken müssen.

Zusätzlich kann eine Messung der Absorption von IR-Strahlung in einem anderen Wellenlängenbereich oder bei einer anderen Wellenlänge durch das Reaktionsgemisch zur Nulllinienkorrektur durchgeführt werden. Eine solche Nulllinienkorrektur kann notwendig sein, um den Einfluss von Intensitätsschwankungen der IR-Strahlung emittierenden Lichtquelle auszugleichen. Der Wellenlängenbereich oder die Wellenlänge werden dafür so ausgewählt, dass die Nitro- und Nitrosoverbindungen in diesem Wellenlängenbereich beziehungsweise bei dieser Wellenlänge keinen oder einen vernachlässigbar kleinen Beitrag an der gemessenen Absorption durch das Reaktionsgemisch haben. Diese Messung ergibt einen Korrekturwert. Die Konzentrationsbestimmung der Nitro- und Nitrosoverbindungen erfolgt in einem Wellenlängenbereich oder bei einer Wellenlänge, bei dem/bei der im Wesentlichen die Nitro- und Nitrosoverbindungen in dem Reaktionsgemisch die IR-Strahlung absorbieren. Die gemessene Absorption wird dann um den Korrekturwert korrigiert, der in dem anderen Wellenlängenbereich/bei der anderen Wellenlänge zur Nulllinienkorrektur gemessen wurde. Die Messung der Absorption durch die Nitro- und Nitrosoverbindungen und der Korrekturwerte kann gleichzeitig durch zwei auf verschiedene Wellenlängenbereiche) eingestellte Photometer kontinuierlich erfolgen. Es kann aber auch mit einem Photometer ein beide Wellenlängenbereiche) umfassendes Spektrum aufgenommen werden oder es können abwechselnd Messungen bei den zwei verschiedenen Wellenlängen durchgeführt werden.

Die Katalysatoren müssen nicht, können aber gemäß der vorliegenden Erfindung aus dem Reaktionsgemisch mit mindestens einem Abtrennverfahren abgetrennt werden, das aus der Gruppe Membranfiltration, Sedimentation und Zentrifugation ausgewählt ist oder mit einem beliebigen anderen, im Stand der Technik bekannten Verfahren. Das Abtrennen eines in einem Reaktionsgemisch enthaltenen Katalysators ist zum Beispiel aus DE-A1 32 45 318, DE-A1 30 40 631 oder WO 03/066571 bekannt. Es ist auch eine Kombination von Abtrennverfahren möglich (zum Beispiel Sedimentation und anschließende Membranfiltration), um eine möglichst vollständige Abtrennung des Katalysators aus dem Reaktionsgemisch zu erreichen, bevor Absorptionsmessungen durchgeführt werden.

Die Messung der Absorption von IR-Strahlung kann bei einem gegenüber dem Umgebungsdruck erhöhten Druck und/oder bei Umgebungsdruck durchgeführt werden. Beispielsweise kann ein Photometer in einem Bereich hinter einem Membranfilter zur Abtrennung des Katalysators angeordnet sein, in dem das aus dem Filter austretende Filtrat unter einem gegenüber dem Umgebungsdruck erhöhten Druck steht. Die Absorptionsmessung kann aber auch in einem Bereich einer Vorrichtung zur Herstellung von aromatischen Aminen angeordnet sein, in dem der Katalysator bereits abgetrennt ist und das Reaktionsgemisch auf Umgebungsdruck entspannt ist.

Besonders bevorzugt wird bei einem Druck gearbeitet, unter dem das Reaktionsgemisch im Reaktor steht. Durch den Bypass strömt das Reaktionsgemisch unter dem Reaktionsdruck aus dem Reaktor. Nach Verschließen der Ventile liegt das Reaktionsgemisch ebenfalls unter diesem Druck vor. Eine vorhergehende separate Abtrennung des Katalysators ist dabei nicht erforderlich, da die Sedimentation im Bypass nach Verschließen der Ventile ausreichend ist.

Die Messung der Absorption von IR-Strahlung durch das Reaktionsgemisch kann in dem aus dem Reaktor entnommenen Hauptstrom oder bevorzugt in einem von dem Hauptstrom abzweigenden Nebenstrom (Bypass) erfolgen.

Für eine Bestimmung geeignete IR-Spektrometer, insbesondere NIR-Spektrometer, sind kommerziell erhältlich, beispielsweise von Bruker GmbH.

Bevorzugt wird mittels eines NIR-Spektrometers und eines geeigneten Rechner gestützten Kalibrationsmodells der Gehalt an Nitroaromaten und Nitrosoaromaten während der Hydrierung online im Hydrierbad bestimmt. Von dem NIR-Spektrometer werden dann z. B. über einen Feldbus Daten zur Ausgabe des Gehalts an Nitroaromaten und Nitrosoaromaten an das Prozessleitsystem übertragen. Aufgrund des festgestellten Gehalts an Nitroaromaten und Nitrosoaromaten ist eine Regelung durch das Prozessleitsystem für die Zuführung von Nitroaromat möglich.

Für die Ausgestaltung der Inline-Messung von Infrarot-, vorzugsweise Nah-Infrarot-Spektren und die Auswertung der gemessenen Spektren mittels eines Rechner-gestützten Matrix-spezifischen Kalibrationsmodells und die Verbindung mit einem Prozessleitsystem kann auf die EP-A-1 445 246 für eine nähere Beschreibung verwiesen werden.

Bevorzugt werden Dinitrotoluol, insbesondere 2,4-Dinitrotoluol oder dessen technische Gemische mit 2,6-Dinitrotoluol zu dem entsprechenden Amin hydriert. Gemäß einer besonders bevorzugten Ausführungsform der vorliegenden Erfindung wird Toluoldiamin durch Hydrierung von Dinitrotoluol hergestellt und eine Messung der Absorption von IR-Strahlung zur Konzentrationsbestimmung im Wesentlichen von in dem Reaktionsgemisch enthaltenem Dinitrotoluol und Aminonitrotoluol durchgeführt. Aminonitrotoluol ist ein bei der Hydrierung von Dinitrotoluol entstehendes Zwischenprodukt. Vorzugsweise wird die Messung der Absorption durch im Wesentlichen Dinitrotoluol und Aminonitrotoluol in dem Reaktionsgemisch bei einer Wellenlänge im Bereich von 600 bis 1365 nm, vorzugsweise bei 650 bis 1200 nm, durchgeführt oder ein Absorptionsspektrum in einem Wellenlängenbereich zwischen 600 und 1365 nm aufgenommen. In dem Bereich von 665 bis 965 nm wird die Strahlung im Wesentlichen durch das in dem Reaktionsgemisch enthaltene Dinitrotoluol und Aminonitrotoluol absorbiert. Die Absorption durch das Toluoldiamin und das in dem Reaktionsgemisch enthaltene Wasser ist in diesem Wellenlängenbereich gering. In einem Wellenlängenbereich von 650 bis 750 nm, bevorzugt bei 700 nm, können Absorptionsmessungen zur Nulllinienkorrektur durchgeführt werden. In diesem Wellenlängenbereich liegt die Absorption durch Dinitrotoluol und Aminonitrotoluol nahezu bei Null.

Das IR-Spektrometer enthält eine IR-Lichtquelle und einen Detektor, zwischen denen ein Behältnis angeordnet ist, durch das das Reaktionsgemisch strömen kann. Der Weg, den das von der Lichtquelle emittierte Licht bei der Absorptionsmessung durch das in dem Behältnis vorhandene Reaktionsgemisch zurücklegt, bevor es aus dem Behältnis wieder austritt und von dem Detektor detektiert wird, ist die so genannte Schichtdicke, die vorzugsweise zwischen 0,5 und 1,5 cm liegt. Die Lichtquelle und der Detektor sind vorzugsweise in einer Durchflusszelle montiert, die mit Reaktionsgemisch führenden Nebenstromleitungen verbunden ist.

Die Lichtquelle strahlt in einem möglichst weiten Spektralbereich (Kontinuumstrahler), falls Absorptionsspektren aufgenommen werden sollen. Für die Messung der Absorption bei einer bestimmten Wellenlänge genügt auch eine Lichtquelle, die mit einem schmalen Spektralbereich strahlt, insbesondere eine monochromatische Lichtquelle. Die Lichtquelle kann auch zwei verschiedene Lampen umfassen, um ein großes Spektrum abzudecken. Ein Monochromator kann zur Zerlegung des Lichts in einzelne Wellenlängen dienen. Der Detektor kann zum Beispiel eine Vakuumphotozelle, einen Photomultiplier oder ein Feld von Silicium-Photodioden enthalten.

Das von dem Detektor detektierte Signal wird gegebenenfalls durch einen Verstärker verstärkt und in einer Auswertungseinheit (zum Beispiel einem Rechner) ausgewertet. Das Ergebnis der aus der gemessenen Absorption bestimmten Konzentration an Nitro- und Nitrosoverbindungen wird zum Beispiel auf einer Anzeige zur Information eines Anwenders angezeigt. Es kann zur Regelung des Amin-Herstellungsverfahrens herangezogen werden oder ein Alarmsignal auslösen, falls eine bestimmte Nitro- und Nitrosoverbindungskonzentration überschritten wird. Ferner kann die Auswerteeinheit so programmiert sein, dass diese eine aufgrund von Gasblasen im Reaktionsgemisch fehlerhafte Messung erkennt und verwirft (d.h. nicht anzeigt, nicht zur Regelung des Verfahrens verwendet oder auch sonst nicht berücksichtigt).

Die Katalysatoren müssen nicht, können aber gemäß der vorliegenden Erfindung aus dem Reaktionsgemisch mit mindestens einem Abtrennverfahren abgetrennt werden. Durch die Online-Überwachung der Konzentration von Nitro- und Nitrosoverbindungen kann die manuelle Probennahme und die Offline-Analytik von Nitro- und Nitrosoaromaten vermieden werden. Eine manuelle Probennahme stellt häufig ein erhebliches Risiko dar, und die Offline-Analytik ist aufwändig und zeitintensiv.

Da bereits DNT-Mengen von über 1000 ppm ein erhebliches Sicherheitsrisiko bezüglich thermischer Zersetzung darstellen und da bereits Mengen oberhalb von 30 und insbesondere oberhalb von 100 ppm zur verstärkten Katalysatordesaktivierung führen, kann durch die Online-Verfolgung der Reaktionsmischung eine (ungewollte) Zunahme von DNT schnell erkannt und gegengesteuert werden. Eine Beschleunigung der Desaktivierung des Katalysators durch eine erhöhte Nitroaromaten-Konzentration und eine entsprechend einsetzende Negativspirale kann damit verhindert werden.

Bevorzugt wird die Dinitrotoluol-Konzentration im flüssigen Produktaustrag aus dem Reaktor auf einen Wert im Bereich von 1 bis 100 Gew.-ppm, bezogen auf das Gesamtgewicht des flüssigen Produktaustrages aus dem Reaktor, weiter bevorzugt auf einen Wert von 2 bis 50 Gew.-ppm, bezogen auf das Gesamtgewicht des flüssigen Produktaustrages aus dem Reaktor und besonders bevorzugt auf einen Wert im Bereich von 3 bis 30 Gew.-ppm, bezogen auf das Gesamtgewicht des flüssigen Produktaustrages aus dem Reaktor, eingestellt.

Möglichkeiten einer erhöhten Nitroaromatenkonzentration entgegenzuwirken sind eine verstärkte Dosierung von Frischkatalysator, eine Reduzierung der kontinuierlich dosierten Menge an Nitroaromaten, eine Erhöhung der Reaktionstemperatur, des Wasserstoffpartialdrucks oder der Intensität der Durchmischung (z. B. Erhöhung der Rührerdrehzahl im Falle eines Rührkesselreaktors). Bevorzugt wird bei geringfügig erhöhten Nitroaromatenkonzentrationen zunächst die Dosierung von Frischkatalysator erhöht und, falls dies nicht ausreicht, zusätzlich die kontinuierlich dosierte Menge an Nitroaromaten reduziert.

Nach einer bevorzugten Ausführungsform der Erfindung wird mittels eines GC und eines geeigneten Rechnergestützten Kalibrationsmodells der Gehalt an Nitroaromaten und Nitrosoaromaten während der Hydrierung online im Hydrierbad bestimmt. Von dem GC werden dann über PLT-typische Schnittstellen, z. B. Feldbus, Daten zur Ausgabe des Gehalts an Nitroaromaten und Nitrosoaromaten an das Prozessleitsystem übertragen. Aufgrund des festgestellten Gehalts an Nitroaromaten und Nitrosoaromaten ist eine Regelung durch das Prozessleitsystem für die Zuführung von Nitroaromat oder Katalysator möglich. Aus der US 5,121,337 und der EP-0 094 876 B1 ist an sich die online Steuerung einer Produktionsanlage zur Regelung verschiedener Polymerisationsparameter bekannt geworden, wobei ein auf der Basis von gemessenen Spektren erstelltes prädikatives Modell eingesetzt wird. Des Weiteren ist in EP 1 445 246 A2 die geeignete Überwachung und/oder Führung eines Nitrierprozesses beschrieben.

Es hat sich gezeigt, dass mithilfe des erfindungsgemäßen Verfahrens bereits kleine Mengen an Dinitrotoluol in einem Hydrierprozess nachweisbar sind. Da DNT-Mengen von über 1000 ppm bereits ein erhebliches Sicherheitsrisiko bezüglich thermischer Zersetzung darstellen und da Mengen oberhalb von 30 und insbesondere oberhalb von 100 ppm zur verstärkten Katalysatordesaktivierung führen, kann durch die Online-Verfolgung der Reaktionsmischung eine Überdosierung von DNT automatisch über das Prozessleitsystem vermieden werden.

Ein wichtiges Maß für die Zuverlässigkeit des Gerätes stellt die Wiederholbarkeit der Analysenergebnisse dar. Zur Bestimmung dieser Messgröße werden wiederholte Analysen (üblicherweise über einen Zeitraum von 8 Stunden) ein und derselben Probe herangezogen. Typische Wiederholbarkeiten in der Prozess-GC sind <+/- 0,5 % für Hauptkomponenten, <+/- 2 % für Spuren. Hierbei ist jedoch zu beachten, dass die Wiederholbarkeit nur Aussagen über das Ausmaß zufälliger Messfehler zulässt. Die Richtigkeit eines gaschromatographisch ermittelten Messwertes kann darüber hinaus auch wesentlich von geräteunabhängigen Faktoren (z. B. Konstruktion der Probenahme, Güte des verwendeten Kalibriermediums, Qualität der eingesetzten Betriebsgase) abhängen.

Die Erfindung ist besonders vorteilhaft, da sie eine deutlich verbesserte Prozessführung ermöglicht. Insbesondere ermöglicht es die Erfindung, die Produktionsanlage kontinuierlich nah am technischen und wirtschaftlichen Optimum zu betreiben. Ein weiterer Vorteil ist die Verbesserung der Arbeitssicherheit.

Das Hydrierverfahren kann in beliebiger geeigneter Weise durchgeführt werden. Für geeignete Verfahren und darin einsetzbare Katalysatoren kann beispielsweise auf DE-A-2 044 657, EP-A-1 138 665, WO 2005/037768, WO 00/35852, DE-A-198 57 409, EP-A-0 124 010 und die prioritätsältere, nicht vorveröffentlichte PCT/EP 2009/067610 bzw. EP 10 162 924.4 verwiesen werden.

Als Reaktoren werden die üblichen und bekannten Hydrierreaktoren verwendet. Beispiele hierfür sind Rührkessel, Wirbelbettreaktoren, monolitische, katalytische Hydrierreaktoren, wie beispielsweise in EP-A 2 1310302 beschrieben, Rohrbündelreaktoren, Blasensäulen, die Packungen enthalten können, oder Schlaufen- oder Kreisstromreaktoren, wie Loop-Venturi-Reaktoren, oder Strahlschlaufenreaktoren mit innerem und äußerem Kreislauf, wie beispielsweise in WO 00/35852, bzw. DE-A-198 57 409 oder WO 03/068724 beschrieben. Auch ein wie in EP-A-0 124 010 beschriebener Reaktor kann eingesetzt werden.

Vorzugsweise werden mit dem erfindungsgemäßen Herstellungsverfahren aromatische Amine durch Hydrierung von Nitroaromaten mit einer oder mehreren Nitrogruppen und 6 bis 18 C-Atomen hergestellt. Die Nitroaromaten sind beispielsweise Nitrobenzol, Dinitrobenzole wie 1,2-, 1,3-, 1,4-Dinitrobenzol, Nitrotoluole, wie o-, m-, p-Nitrotoluol, Dinitrotoluole wie 2,4-, 2,6-, 2,3-, 3,4-, 2,5-Dinitrotoluol, 2,4,6-Trinitrotoluol, Nitroxylole, wie 1,2-Dimethyl-3-, 1,2-Dimethyl-4-, 1,4-Dimethyl-2-, 1,3-Dimethyl-2-, 2,4-Dimethyl-1- und 1,3-Dimethyl-5-nitrobenzol, Nitronaphthaline, wie 1-, 2-Nitronaphthalin, 1,5- und 1,8-Dinitronaphthalin, Chlornitrobenzole, wie 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1- und 1,2-Dichlor-3-nitrobenzol, Chlornitrotoluole, wie 4-Chlor-2-, 4-Chlor-3-, 2-Chlor-4- und 2-Chlor-6-nitrotoluol, Nitroaniline, wie o-, m-, p-Nitroanilin sowie beliebige Gemische aus 2 oder mehreren der genannten Nitroverbindungen.

Die Hydrierung wird im erfindungsgemäßen Verfahren vorzugsweise unter einem Druck im Bereich von 5 bis 50 bar, bevorzugt bei einem Druck im Bereich von 10 bis 40 bar, besonders bevorzugt bei einem Druck im Bereich von 20 bis 30 bar, durchgeführt. Die Betriebstemperatur, bei der die Hydrierung von Dinitrotoluol zu Toluylendiamin durchgeführt wird, liegt allgemein im Bereich von 50 bis 250 °C, bevorzugt im Bereich von 80 bis 200 °C, besonders bevorzugt im Bereich von 105 bis 130 °C.

Gemäß einer weiteren bevorzugten Ausführungsform der Erfindung wird das erfindungsgemäße Verfahren so betrieben, dass auch die Konzentration des teilhydrierten Zwischenproduktes Aminonitrotoluol, im Folgenden abgekürzt als ANT bezeichnet, kontrolliert wird, und zwar im Bereich zwischen dem Reaktor und der Produktabtrenneinheit auf eine ANT-Konzentration im Bereich von 0 bis 2.000 ppm, bevorzugt auf eine ANT-Konzentration im Bereich von 0,5 bis 1.000 ppm, besonders bevorzugt eine ANT-Konzentration im Bereich von 1 bis 200 ppm, eingestellt wird. Die Einstellung der Konzentration dieses teilhydrierten Zwischenprodukts in den genannten Bereichen ermöglicht zum einen eine weitere Reduzierung der Katalysatordesaktivierung und zum anderen eine weitere Reduzierung der Nebenproduktbildung. In der Regel ist der Verlauf der Konzentrationen von DNT und ANT weitgehend parallel.

Zur Herstellung von TDA kommt eine Vielzahl von Katalysatoren in Frage.

Gemäß einer ersten allgemeinen Ausführungsform der Erfindung wird das erfindungsgemäße Verfahren, wie oben ausgeführt, in Gegenwart eines Katalysators, bevorzugt eines Nickel enthaltenden Katalysators, insbesondere in Gegenwart eines Trägerkatalysators, der als Aktivkomponente Nickel allein oder zusammen mit mindestens einem Metall der I., V., VI. und/oder VIII. Nebengruppe des Periodensystems aufweist, durchgeführt. Die erfindungsgemäß verwendeten Katalysatoren können technisch durch Aufbringen von Nickel und gegebenenfalls mindestens einem der oben genannten zusätzlichen Metalle auf einen geeigneten Träger hergestellt werden.

Gemäß einer bevorzugten Ausführungsform der Erfindung weist der Katalysator einen Nickelgehalt zwischen 0,1 und 99 Gew.-%, bevorzugt zwischen 1 und 90 Gew.-%, besonders bevorzugt zwischen 25 und 85 Gew.-% und ganz besonders bevorzugt zwischen 60 und 80 Gew.-%, bezogen auf das Gesamtgewicht des Katalysators auf.

Vorzugsweise werden als Metalle der I., II., V., VI. und/oder VIII. Nebengruppe des Periodensystems Palladium, Platin, Rhodium, Eisen, Kobalt, Zink, Chrom, Vanadium, Kupfer, Silber oder ein Gemisch aus zwei oder mehreren davon verwendet.

Gemäß einer bevorzugten Ausführungsform der Erfindung enthält der Katalysator Nickel und Platin. Gemäß einer weiteren bevorzugten Ausführungsform der Erfindung enthält der Katalysator Nickel und Aluminium; gemäß einer weiteren besonders bevorzugten Ausführungsform enthält der Katalysator Nickel, Palladium und Eisen.

Als Trägermaterialien werden vorzugsweise Aktivkohle, Ruß, Graphit, oder oxidische Trägerkomponenten wie Siliziumdioxid, Siliziumcarbid, Kieselgur, Aluminiumoxid, Magnesiumoxid, Titandioxid, Zirkoniumdioxid und/oder Hafniumdioxid oder ein Gemisch aus zwei oder mehr davon, besonders bevorzugt Zirkoniumdioxid, $ZrO_2$, $HfO_2$ und/oder $SiO_2$, $ZrO_2$ und/oder $SiO_2$, $ZrO_2$, $HfO_2$, verwendet.

Die verwendeten Träger sind vorzugsweise mesoporös und besitzen einen mittleren Porendurchmesser von 35 bis 50 nm und eine spezifische Oberfläche von 50 bis 250 $m^2/g$. Die Oberfläche des Trägers wird bestimmt nach dem BET-Verfahren durch $N_2$-Adsorption, insbesondere nach DIN 66131. Die Bestimmung des mittleren Porendurchmessers und der Porengrößenverteilung erfolgt durch Hg-Porosimetrie, insbesondere nach DIN 66133.

Das Aufbringen von Nickel und gegebenenfalls mindestens eines weiteren Metalls kann durch die üblichen geeigneten Verfahren, die dem Fachmann auf dem Gebiet der Katalysatortechnik bekannt sind, erreicht werden. Die mit dem Metall bzw. Metallsalz beschichteten, durch gemeinsame Fällung beschichteten oder getränkten Träger werden anschließend nach bekannten Verfahren getrocknet und kalziniert. Darauf folgend werden die beschichteten Träger durch Behandlung derselben in einem Gasstrom, der freien Wasserstoff enthält, aktiviert. Diese Aktivierung findet zumeist bei Temperaturen im Bereich von 30 bis 600 °C, vorzugsweise im Bereich von 80 bis 150 °C und insbesondere bevorzugt bei 100 °C statt. Der Gasstrom besteht vorzugsweise aus 50 bis 100 Vol.-% Wasserstoff und 0 bis 50 Vol.-% Stickstoff. Der für den erfindungsgemäßen Einsatz hergestellte Katalysator weist nach einstündiger Reduktion bei 100 °C einen Reduktionsgrad von mindestens 70 % auf.

Die so erhaltenen Trägerkatalysatoren besitzen im Allgemeinen eine Nickel-Metalloberfläche von ungefähr 10 bis ungefähr 50 $m^2/g$, vorzugsweise ungefähr 20 bis ungefähr 60 $m^2/g$. Der Nickelgehalt der im Rahmen des erfindungsgemäßen Verfahrens eingesetzten Katalysatoren liegt allgemein im Bereich von 0,1 bis 99 Gew.-%, bevorzugt im Bereich von 1 bis 90 Gew.-%, besonders bevorzugt im Bereich von 25 bis 85 Gew.-%, bezogen auf das Gesamtgewicht der eingesetzten Katalysatoren.

Geeignete Katalysatoren dieser Ausführungsform sind z. B. in den Druckschriften EP 1 161 297 A1 und EP 1 165 231 A1 beschrieben.

Gemäß einer zweiten Ausführungsform der Erfindung werden im Rahmen des erfindungsgemäßen Verfahrens aktivierte Nickelkatalysatoren, wie beispielsweise in der WO 2008/145179 A1 beschrieben, eingesetzt. Demgemäß werden gemäß einer bevorzugten Ausführungsform der Erfindung aktivierte Nickelkatalysatoren auf Basis einer Ni/Al-Legierung, die ein oder mehrere Metalle, ausgewählt aus der Gruppe Mg, Ce, Ti, V, Nb, Cr, W, Mn, Re, Fe, Ru, Co, Rh, Ir, Pt, Cu, Ag, Au und Bi enthalten können, eingesetzt. Der Dotierungsgrad liegt dabei im Bereich von 0,05 Gew.-% bis 20 Gew.-% für jedes Dotierungselement. Die mittlere Partikelgröße der eingesetzten Katalysatoren ist < 25 μm.

Gemäß einer dritten Ausführungsform der Erfindung werden im Rahmen des erfindungsgemäßen Verfahrens Katalysatoren eingesetzt, die beispielsweise in der WO 2008/138784 A1 beschrieben sind. Gegenstand der Erfindung ist somit gemäß einer bevorzugten Ausführungsform der Erfindung weiterhin die Verwendung von Hydrierkatalysatoren, enthaltend als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger, zur Herstellung von aromatischen Aminen durch katalytische Hydrierung der entsprechenden Nitroverbindungen, insbesondere zur Herstellung von Toluylendiamin durch Hydrierung von Dinitrotoluol. Das zusätzliche Element ist vorzugsweise ausgewählt aus der Gruppe, enthaltend Kobalt, Eisen, Vanadium, Bismut und Zinn.

Als Träger für die Katalysatoren können die hierfür üblichen und bekannten Materialien eingesetzt werden.

Vorzugsweise werden Aktivkohle, Ruß, Graphit oder Metalloxide, bevorzugt hydrothermal stabile Metalloxide wie z. B. $ZrO_2$, $TiO_2$, $Al_2O_3$ eingesetzt. Bei Graphit sind die HSAG (High Surface Area Graphite) mit einer Oberfläche von 50 bis 300 $m^2/g$ besonders bevorzugt. Besonders bevorzugt sind Aktivkohlen, insbesondere physikalisch oder chemisch aktivierte Aktivkohlen, oder Ruße, wie Acetylenruß.

Die erfindungsgemäßen Hydrierkatalysatoren enthalten vorzugsweise 0,5 bis 5 Gew.-% Palladium, 10 bis 20 Gew.-% Nickel und 0,5 bis 5 Gew.-% des Zusatzelementes.

Im Rahmen des erfindungsgemäßen Verfahrens werden 2,4-DNT oder dessen technische Gemische weiterhin enthaltend 2,6-DNT, wobei diese Gemische vorzugsweise bis zu 35 Gew.-%, bezogen auf das Gesamtgemisch, an 2,6-DNT mit Anteilen von 1 bis 4 % an vicinalem DNT und 0,5 bis 1,5 % an 2,5- und 3,5-DNT aufweisen, zu dem entsprechenden Amin hydriert. Häufig werden die DNT-Isomeren im bei der zweifachen Nitrierung von Toluol anfallenden Isomerenverhältnis eingesetzt.

In dem erfindungsgemäßen Verfahren kann das 2,4-DNT oder das 2,4-DNT/2,6-DNT-Gemisch in reiner Form, als Mischung mit Wasser, als Mischung mit Wasser und einem alkoholischen Lösungsmittel oder als Mischung mit Wasser, einem alkoholischen Lösungsmittel und einem Katalysator-reaktivierenden Zusatz eingesetzt werden. Ebenso können Katalysator, Wasser und/oder alkoholische Lösungsmittel oder Gemische hiervon gemeinsam oder getrennt zusätzlich zum DNT separat dosiert werden.

Wie sich aus dem oben Gesagten ergibt, kann beim erfindungsgemäßen Verfahren die Hydrierung unter Abwesenheit oder in Gegenwart eines alkoholischen Lösungsmittels und eines Katalysator-aktivierenden Zusatzes durchgeführt werden.

Sofern ein alkoholisches Lösungsmittel und ein Katalysator-reaktivierender Zusatz verwendet werden, können selbstverständlich auch Gemische aus zwei oder mehr davon zugesetzt werden.

Als alkoholische Lösungsmittel werden niedere aliphatische Alkohole mit 1 bis 6 Kohlenstoffatomen, vorzugsweise Methanol, Ethanol oder Propanol einzeln oder ein Gemisch aus zwei oder mehreren davon verwendet.

Als Katalysator-aktivierende Zusätze werden vorzugsweise aprotische Lösungsmittel, insbesondere Aceton, Dimethylformamid, Dioxan oder Tetrahydrofuran oder ein Gemisch aus zwei oder mehreren davon eingesetzt.

Die Menge der eingesetzten alkoholischen Lösungsmittel und der Katalysator-reaktivierenden Zusätze ist im Rahmen des erfindungsgemäßen Verfahrens nicht in besonderer Weise beschränkt und kann je nach Bedarf vom Fachmann frei gewählt werden.

Gemäß einer bevorzugten Ausführungsform der Erfindung wird die Hydrierung in einem Dreiphasengemisch aus wasserstoffhaltiger Gasphase, suspendiertem Katalysator und Flüssigphase enthaltend 0 bis 40 Vol.-% eines Alkohols, 10 bis 60 Vol.-% Wasser und 20 bis 70 Vol.-% TDA, wie vorstehend definiert, durchgeführt. Der Katalysatorgehalt beträgt dabei etwa 0,1 bis 15 Gew.-%, bevorzugt 2 bis 8 Gew.-%, bezogen auf das Gesamtgewicht des eingesetzten Dreiphasengemisches.

Das Ergebnis der bestimmten Konzentration an Nitro- und Nitrosoverbindungen wird zum Beispiel auf einer Anzeige zur Information eines Anwenders angezeigt. Es kann zur Regelung des Amin-Herstellungsverfahrens herangezogen werden oder ein Alarmsignal auslösen, falls eine bestimmte Nitro- und Nitrosoverbindungskonzentration überschritten wird.

Mit der erfindungsgemäßen Vorrichtung ist eine automatische quasikontinuierliche Online-Messung der Konzentration der Nitro- und Nitrosoverbindungen in dem Reaktionsgemisch möglich. Durch das auf der Konzentrationsmessung basierende, bereits beschriebene Regelungsverfahren kann in vorteilhafter Weise der Katalysatorverbrauch optimiert und damit reduziert werden.

Anhand der Beispiele und der Zeichnungen wird die Erfindung nachstehend näher erläutert.

Es zeigen:

Fig. 1 ein Flussdiagramm einer Ausführungsform eines erfindungsgemäßen Verfahrens zur Bestimmung der Nitroaromatenkonzentration in verschiedenen Hydrierbädern,

Fig. 2 ein Blockdiagramm einer Hydrieranlage mit einer Ausführungsform der erfindungsgemäßen Regelung,

Fig. 3 - Fig. 7 zeigen Chromatogramme des Reaktionsgemisches mit den verschiedenen Isomeren; analysiert nach der Methode 3a, 3b und 3c:

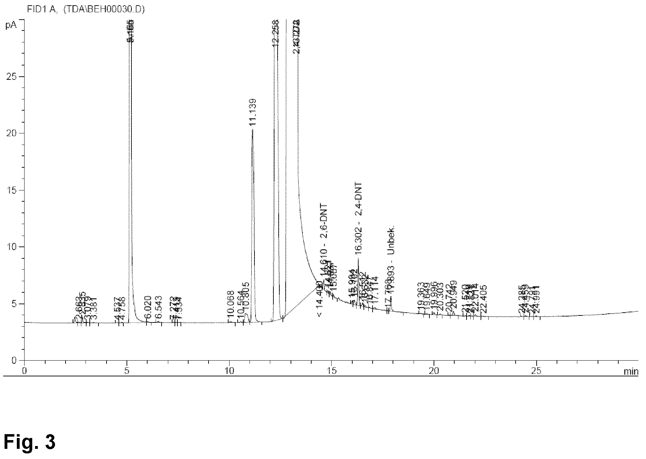

Fig. 3 (Analytik gemäß Methode 3a)

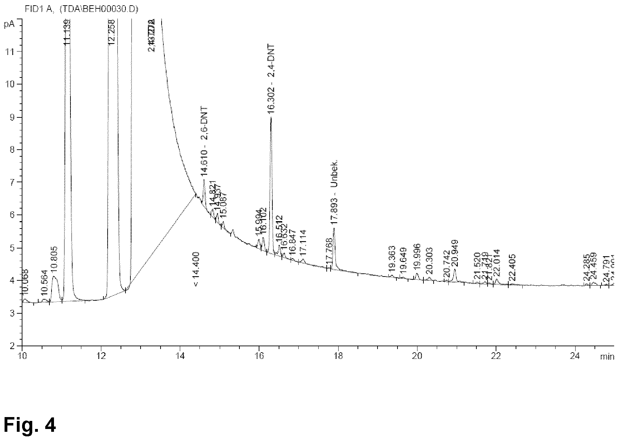

Fig. 4 (Analytik gemäß Methode 3a - Ausschnittsvergrößerung)

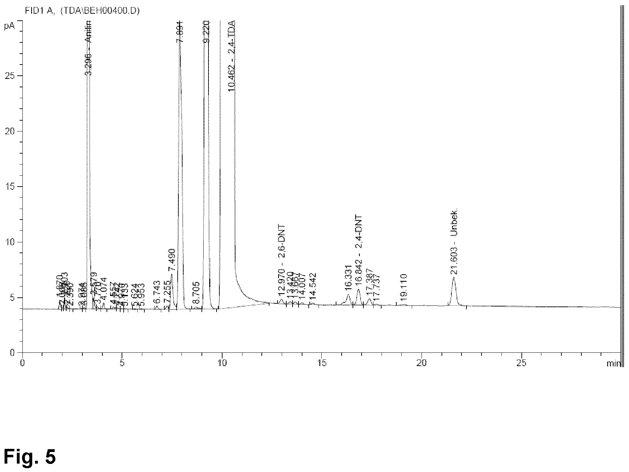

Fig. 5 (Analytik gemäß Methode 3b)

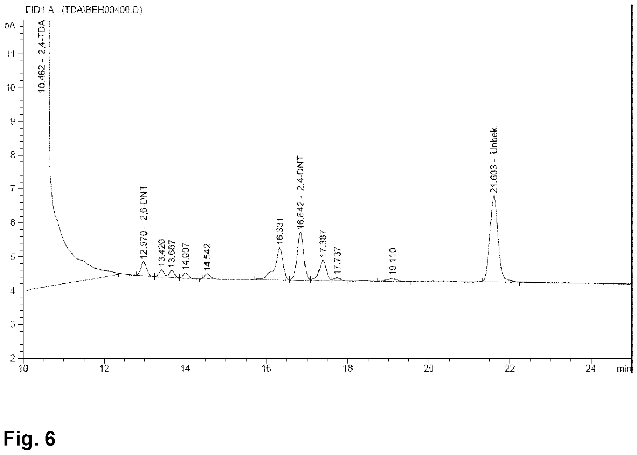

Fig. 6 (Analytik gemäß Methode 3b - Ausschnittsvergrößerung)

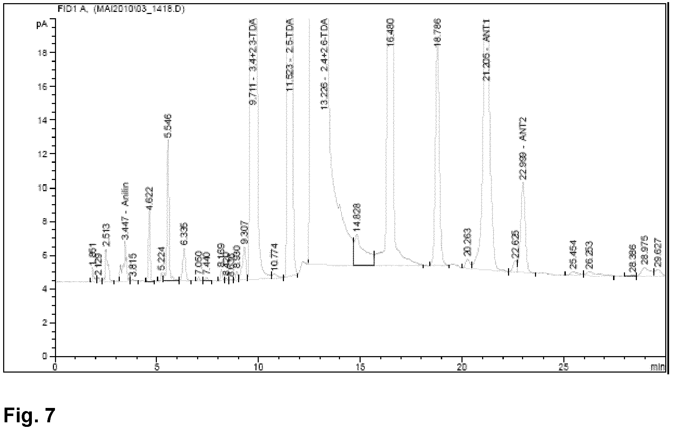

Fig. 7 (Analytik gemäß Methode 3c)

Fig. 8 eine schematische Darstellung einer Anlage zur Durchführung des erfindungsgemäßen Verfahrens,

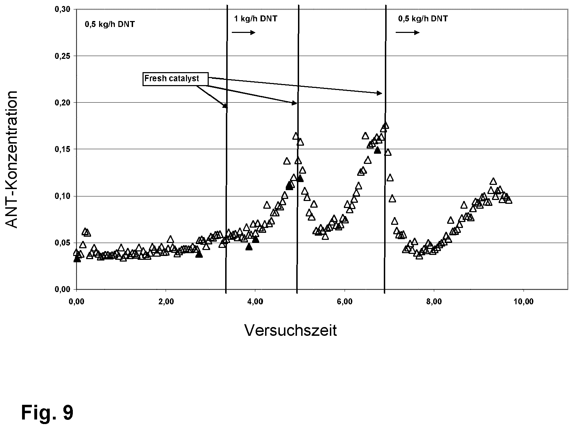

Fig. 9 zeigt mittels Online-GC gemessene ANT-Konzentrationen in Gew.-% (weiße Symbole) und mittels Offline-GC ermittelte ANT-Konzentrationen in Gew.-% (schwarze Symbole) als Funktion der Versuchszeit in Tagen.

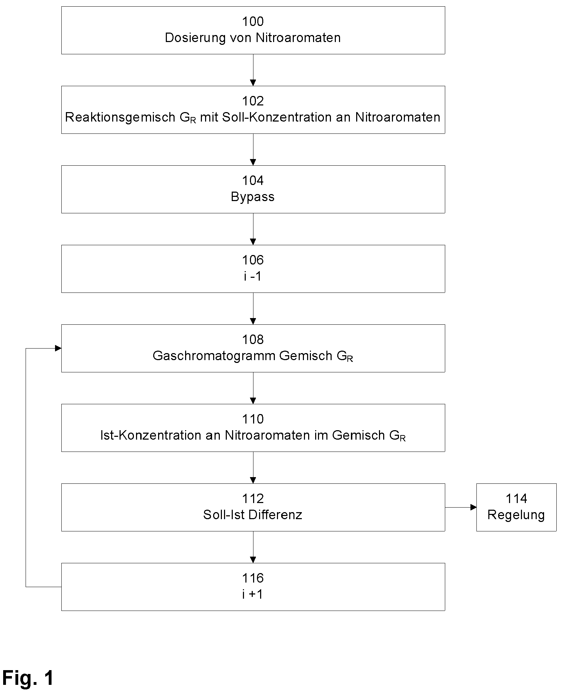

In dem Schritt 100 in Figur 1 erfolgt die Dosierung von Nitroaromaten in das Hydrierbad 102, welches das Reaktionsgemisch $G_R$, bestehend aus dem Katalysator, der Mischung aus aromatischem Amin und Wasser, sowie eine Atmosphäre von Wasserstoff enthält. Das Reaktionsgemisch enthält je nach Aktivität des Katalysators und Dosiergeschwindigkeit geringe Mengen an Nitroaromaten, bzw. Aminonitroaromaten, welche eine bestimmte Soll-Konzentration nicht überschreiten sollen.

Über einen angeschlossenen Bypass 104 wird ein Teil des Hydrierbades abgezweigt. In dem Schritt 106 wird der Index i initialisiert. In dem Schritt 108 wird online das Chromatogramm des Reaktionsgemisches $G_R$ aufgenommen. Dies erfolgt beispielsweise durch eine online Prozess-GC-Messung. Daraus resultiert in dem Schritt 110 die Ist-Konzentration des Gemisches $G_R$. In dem Schritt 112 wird aus den Soll- und den Ist-Werten der Konzentrationen an Nitroaromaten des Reaktionsgemisches $G_R$ die Differenz gebildet. Auf der Basis dieses Differenzwertes erfolgt in dem Schritt 114 eine Nachregelung der Nitroaromatendosierung. In dem Schritt 116 wird der Index i inkrementiert. Anschließend wird erneut ein Chromatogramm in dem Schritt 108 gemessen. Dieser Ablauf wiederholt sich solange, bis die Ist-Nitroaromatenkonzentration für alle gemessenen Reaktionsgemische $G_R$ zwischen 3 und 30 ppm ist. Danach wird der Index i zurückgesetzt, so dass kontinuierlich innerhalb relativ kurzer Zeitabstände, z. B. weniger Minuten die Ist-Nitroaromatenkonzentration sämtlicher gemessenen Reaktionsgemische $G_R$ bestimmt werden und eine entsprechende Nachregelung zeitnah erfolgen kann.

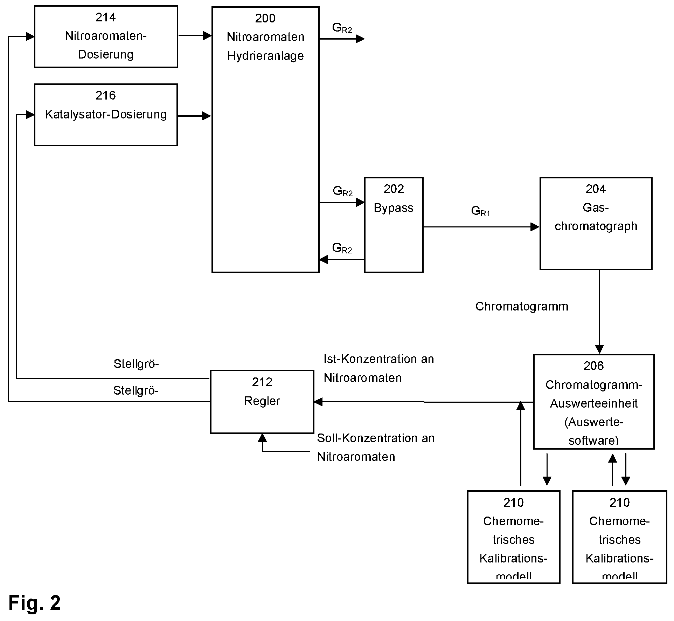

Fig. 2 zeigt eine Hydrieranlage für Nitroaromaten 200 zur Herstellung von Gemischen aus aromatischen Aminen und Wasser $G_{R2}$. Die Probenahme, realisiert durch einen mit einem Teil des Reaktionsgemisches $G_{R2}$ stetig durchströmten Bypass 202 mit Flüssigdosierventil, kann im Bereich zwischen Reaktor und Produktabtrenneinheit (bevorzugt mit Filter) oder nach der Produktabtrenneinheit (bevorzugt Querstromfilter) im Produktstrom erfolgen. Durch zwei Ventile kann der Bypass vom Produktstrom abgetrennt werden (z. B. für Wartungsarbeiten am Gaschromatographen). Die Probenahme (Gaschromatograph) 204 beinhaltet ein druck- und temperaturstabiles Probenahmeventil.

Als Ergebnis der Analyse des gemessenen Chromatogramms erhält man nach Chromatogrammanalyse 206 und/oder Auswertung mittels chemometrischen Kalibrationsmodells 210, welches die Ergebnisse entweder zurück an die Auswerteeinheit oder direkt zum Regler 212 übergibt, die Ist-Konzentration der Reaktionsmischung $G_{R1}$. Diese Ist-Konzentration an Nitroaromaten wird auf einen Regler 212 übertragen sowie auch die Soll-Konzentration an Nitroaromaten des Reaktionsgemisches $G_{R1}$. Aus einer Abweichung zwischen der Ist-Konzentration und der Soll-Konzentration ermittelt der Regler 212 eine Stellgröße für die Nachregelung der Nitroaromatendosierung 214 und/oder die Dosierung des Katalysators 216 der Nitroaromaten Hydrieranlage 200.

Der Regler 212 kann durch ein Prozessleitsystem der Nitroaromaten Hydrieranlage 200 realisiert werden. Alternativ ist es auch möglich, die Messergebnisse beispielsweise auf eine Anzeigeeinheit einer Bedienkonsole der Nitroaromaten Hydrieranlage 200 anzuzeigen, so dass die Nitroaromaten-Dosierung 214 und/oder die Katalysatordosierung 216 manuell nachgeregelt werden kann/können.

Die Figur 8 zeigt die schematische Darstellung einer Anlage zur Durchführung des erfindungsgemäßen Verfahrens.

Der Reaktor 1 ist mit einer in seinem oberen Bereich, nach unten ausgerichteten Treibstrahldüse 2 ausgestattet, über die das Reaktionsgemisch über eine externe Schlaufe 3 in den Reaktor eingedüst wird. Unterhalb der Treibstrahldüse 2 ist ein zentraler, in Reaktorlängsrichtung angeordnetes Einsteckrohr 4 angeordnet und unterhalb des Einsteckrohrs 4 eine Prallplatte 5. Im Innenraum des Reaktors 1 befindet sich ein Fieldrohr-Wärmetauscher 6. Dinitrotoluol, DNT, wird in der in der Figur dargestellten bevorzugten Ausführungsvariante über einen Ringspalt an der Außenwand der als Zweistrahldüse ausgebildeten Treibstrahldüse 2 in den Gasraum oberhalb des Flüssigkeitsspiegels in den Reaktor 1 zugeführt.

Wasserstoff $H_2$, wird in den unteren Bereich des Reaktors 1, in der in der Figur dargestellten bevorzugten Ausführungsvariante, über einen Ringverteiler, und zusätzlich in die externe Schlaufe 3, in der Nähe des Abzugs des Reaktionsgemisches aus dem Reaktorsumpf, eingedüst. Das Reaktionsgemisch wird in der externen Schlaufe über eine Pumpe P, die so ausgelegt ist, dass sie bis zu 20 Vol.-% Gas fördern kann, in der in der Figur dargestellten bevorzugten Ausführungsform über einen Querstromfilter 7 zur Katalysatorabtrennung geleitet. Das Reaktionsgemisch wird anschließend über einen in der externen Schlaufe angeordneten Wärmetauscher W geleitet, der bevorzugt als Rohrbündelwärmetauscher ausgebildet ist. Aus dem in der externen Schlaufe angeordneten Wärmetauscher W wird Dampf über Leitung 8 abgezogen und mit Dampf über Leitung 9 aus den Fieldrohren, die mit Wasser, $H_2O$, gespeist werden, vereinigt, einem Abscheider 10 zugeführt und als Dampf mit 4 bar Überdruck über Leitung 11 abgezogen.

Die Probenahme für die Online-Gaschromatographie kann sich in einem Bypass des flüssigen Produktaustrags zwischen Reaktor 1 und Produktabtrenneinheit 7 (Querstromfilter), vorzugsweise mit einem zusätzlichen Filter (z. B.

Membranfilter) befinden (entspricht Option A) oder in einem Bypass des flüssigen Produktaustrags hinter der Produktabtrenneinheit 7 (entspricht Option B).

Die Erfindung wird durch die nachstehenden Beispiele näher erläutert.

**Beispiele**

**Beispiel 1**

Es wird ein zylinderförmiger Schlaufenreaktor mit einem durch zwei in Reihe geschalteten Kreiselpumpen angetriebenen, in einer zentral am Reaktorkopf angeordneten Treibstrahldüse mündenden externen Kreislauf, einem konzentrischen Einsteckrohr sowie einer Prallplatte im unteren Reaktorteil zur Umlenkung der Schlaufenströmung (interner Kreislauf) eingesetzt (zum Funktionsprinzip vgl. WO 2000/35852 A1). Das Reaktionsvolumen des Reaktors beträgt rund 14 m$^3$. Der Reaktor ist zur Abfuhr der Reaktionswärme mit einem Rohrbündel aus parallel geschalteten Fieldrohren versehen. Die Menge des in die Fieldrohre eingespeisten Kühlwassers wird so eingestellt, dass die Temperatur im Reaktor bei ca. 120 °C gehalten wird. Zur Aufrechterhaltung der Schlaufenströmung wird im externen Produktkreislauf ein Volumenstrom von 600 m$^3$/h umgewälzt, wodurch sich über die Treibstrahldüse ein Druckverlust von ca. 2,5 bar einstellt. Der Reaktor enthält ca. 12 m$^3$ eines flüssigen Hydrierbads. Dieses besteht im Wesentlichen aus einer Mischung von TDA und Wasser im Massenverhältnis von 0,58 : 0,42, in welcher ca. 5 Gew.-% eines auf $SiO_2$ und $ZrO_2$ geträgerten metallischen Ni-Katalysators (hergestellt nach Beispiel 2 der EP 1 161 297 und mittels einer Rührwerksmühle zerkleinert; hierbei bestehen 10 Vol.-% des Katalysators aus Partikeln mit einem Durchmesser ≤ ca. 5 $\mu$m, 50 Vol.-% sind ≤ ca. 10 $\mu$m, und 90 Vol.-% ≤ ca. 15 $\mu$m, gemessen mittels Laserbeugung (Malvern Mastersizer S) nach Aufrühren in Wasser) suspendiert und außerdem Wasserstoff gelöst ist. Der Flüssigkeitsspiegel befindet sich hierbei knapp unterhalb der Mündung der Treibstrahldüse. Darüber befinden sich ca. 2 m$^3$ einer Gasatmosphäre, deren Wasserstoffgehalt durch kontinuierliche Ausschleusung eines geringen Abgasstroms auf 90 bis 95 Vol.-% (neben Inertgasen wie zum Beispiel $N_2$) eingestellt wird. Mit einer Membrankolbendosierpumpe wird in den Gasraum des Reaktors 7,5 t/h geschmolzenes und auf ca. 80 °C temperiertes DNT, bestehend aus einer Mischung der 2,4- und 2,6-DNT-Isomeren im Verhältnis von ca. 80 : 20, sowie ca. 5 % der übrigen DNT-Isomeren und Spuren von Mononitrotoluol eingedüst. Durch gleichzeitiges Einleiten von ca. 0,5 t/h Wasserstoff (verdünnt mit ca. 2 kg/h $N_2$) wird im Reaktor ein Druck von 25 bar eingestellt. 95 % des Wasserstoffs werden über einen Düsenring oberhalb der Prallplatte und 5 % am Reaktorausgang jeweils ins Hydrierbad dosiert. Die Reaktion verläuft unter weitgehend isothermen Bedingungen: Im gesamten Reaktor bewegt sich die Reaktionstemperatur konstant zwischen 116 und 126 °C. Zusätzlich werden über eine Membranpumpe ebenfalls kontinuierlich 625 kg/h einer Suspension des oben genannten Katalysators in Wasser (teilweise im Aufarbeitungsteil aus dem Hydrierprodukt abgetrennt) dosiert. Die Menge des enthaltenen Katalysators in dieser Suspension wird hierbei zur Einstellung der DNT-Konzentration gezielt zwischen 0 und 5 kg/h variiert und beträgt im Mittel etwa 1 kg/h.

Um den Flüssigkeitsstand im Reaktor konstant zu halten, wird dem externen Produktkreislauf auf der Druckseite der 2. Kreiselpumpe kontinuierlich eine entsprechende Menge an Hydrierprodukt entnommen und in einen Lamellenklärer mit ca. 50 m$^3$ Flüssigkeits- und ca. 10 m$^3$ Gasvolumen geleitet. In dessen unterem Bereich kann sich der Katalysator aufkonzentrieren. 18 Nm$^3$/h einer entsprechend eingedickten Suspension werden dann auf die Saugseite der 1. Kreiselpumpe zurückgeführt. Gleichzeitig werden dem Lamellenklärer über einen Überlauf ca. 8,6 t/h Hydrierprodukt entnommen. Dieses enthält ca. 4,9 t/h TDA (mit einer Isomerenverteilung entsprechend der des eingesetzten DNT), ca. 0,1 t/h Leicht- und Schwersieder (im Verhältnis von etwa 20 : 80) sowie ca. 3,6 t/h Wasser und bis zu etwa 1 kg/h Katalysator (v. a. Feinanteil). Das Hydrierprodukt gelangt über eine Druckreduzierung ebenso wie die Hydrierprodukte anderer Reaktoren in einen gemeinsamen Zwischenbehälter und wird von diesem kontinuierlich der destillativen Aufarbeitung zugeführt. Die produktberührenden Teile sind teilweise aus Schwarzstahl (i. d. R. St37) und teilweise aus Edelstahl (1.4571) gefertigt.

Zur Bestimmung des Gehalts an DNT und ANT im Hydrierbad wird aus der Leitung vom externen Produktkreislauf des Schlaufenreaktors zum Lamellenklärer über einen Bypass mit Filtermembran kontinuierlich ein feststofffreier Filtratstrom geleitet, aus dem mittels eines Flüssigdosierventils im Abstand von ca. 1 Stunde eine Online-Probenahme erfolgt. Mittels Online-Gaschromatographie wird so die Konzentration der enthaltenen Nitroverbindungen bestimmt. Durch Anpassung der Katalysatorkonzentration in der dem Reaktor zugeführten wässrigen Suspension (s. o.) wird die DNT-Konzentration zwischen 3 und 30 ppm und die ANT-Konzentration zwischen 1 und 200 ppm eingestellt.

Der Reaktor kann unter den genannten Bedingungen über 3 Monate hinweg ohne nennenswerte Unterbrechungen betrieben werden. In dieser Zeit ist keine nennenswerte Katalysatordesaktivierung zu beobachten.

**Beispiel 2**

Es wird ein Rührkesselreaktor (Durchmesser: 2,8 m, Höhe: 4,7 m, Volumen: 23 m$^3$, Material: St37) mit innen im Bereich des Reaktormantels filigran befestigten Kühlschlangen und einem doppelten Schrägblattrührer verwendet:

Der größere obere Schrägblattkranz drückt das Hydrierbad im Reaktorinneren nach unten, welches dann entlang der Kühlschlangen wieder nach oben strömt; der untere Schrägblattkranz hingegen saugt die von dem hier ebenfalls verwendeten Lamellenklärer zurückfließende eingedickte Suspension an und drückt sie nach oben in die durch den oberen Kranz erzeugte Strömung. Die Menge des in die Kühlschlangen eingespeisten Kühlwassers wird so eingestellt, dass die Temperatur im Reaktor zwischen 116 und 126 °C gehalten wird. Der Reaktor enthält ca. 18 $m^3$ eines flüssigen Hydrierbads. Dieses besteht im Wesentlichen aus einer Mischung von TDA und Wasser im Massenverhältnis von 0,58 : 0,42, in welcher ca. 5 Gew.-% des in Beispiel 1 genannten, auf $SiO_2$ und $ZrO_2$ geträgerten metallischen Ni-Katalysators suspendiert und außerdem Wasserstoff gelöst sind. Oberhalb des Flüssigkeitsspiegels befinden sich ca. 5 $m^3$ einer Gasatmosphäre, deren Wasserstoffgehalt durch kontinuierliche Ausschleusung eines geringen Abgasstroms auf 90 bis 99 Vol.-% (neben Inertgasen wie z. B. $N_2$) eingestellt wird.

Mit einer Membrankolbendosierpumpe werden 5 t/h geschmolzenes und auf ca. 80 °C temperiertes DNT, bestehend aus einer Mischung der 2,4- und 2,6-DNT-Isomeren im Verhältnis von ca. 80 : 20, sowie ca. 5 % der übrigen DNT-Isomeren und Spuren von Mononitrotoluol in einen zum Gasraum hin offenen Trichter dosiert, welcher das DNT über eine Leitung nach unten und zwischen den Schrägblattkränzen ins Hydrierbad leitet. Durch gleichzeitiges Einleiten von ca. 330 kg/h Wasserstoff (verdünnt mit ca. 2 kg/h $N_2$) wird im Reaktor ein Druck von 25 bar eingestellt. Der Wasserstoff wird über einen zwischen den beiden Schrägblattrührerkränzen zentrisch angebrachten und dort filigran befestigten Düsenring ins Hydrierbad dosiert. Die Reaktion verläuft unter weitgehend isothermen Bedingungen. Zusätzlich werden über eine Membranpumpe ebenfalls kontinuierlich 435 kg/h einer Suspension des oben genannten Katalysators in Wasser (teilweise im Aufarbeitungsteil aus dem Hydrierprodukt abgetrennt) dosiert. Die Menge des enthaltenen Katalysators in dieser Suspension wird hierbei zur Einstellung der DNT-Konzentration gezielt zwischen 0 und 5 kg/h variiert und beträgt im Mittel knapp 1 kg/h.

Um den Flüssigkeitsstand im Reaktor konstant zu halten, wird über einen Überlauf kontinuierlich eine entsprechende Menge an Hydrierprodukt entnommen und in einen Lamellenklärer mit ca. 16 $m^3$ Flüssigkeits- und ca. 4 $m^3$ Gasvolumen geleitet. In dessen unterem Bereich kann sich der Katalysator aufkonzentrieren. Ca. 30 $Nm^3$/h einer entsprechend eingedickten Suspension werden dann mit Hilfe des unteren Kranzes des Schrägblattrührers in den Rührkessel zurückgesaugt. Gleichzeitig werden dem Lamellenklären über einen Überlauf ca. 5,8 t/h Hydrierprodukt entnommen. Dieses enthält ca. 3,3 t/h TDA (mit einer Isomerenverteilung entsprechend der des eingesetzten DNT), ca. 0,07 t/h Leicht- und Schwersieder (im Verhältnis von etwa 10 : 60) sowie ca. 2,4 t/h Wasser und bis zu 1 kg/h Katalysator (v. a. Feinanteil). Das Hydrierprodukt gelangt über eine Druckreduzierung ebenso wie die Hydrierprodukte anderer Reaktoren in einen gemeinsamen Zwischenbehälter und wird von diesem kontinuierlich der destillativen Aufarbeitung zugeführt.

Zur Bestimmung des Gehalts an DNT und ANT im Hydrierbad wird aus der Leitung nach dem Lamellenklärer über einen Bypass kontinuierlich ein feststofffreier Filtratstrom geleitet, aus dem mittels eines Flüssigdosierventils im Abstand von ca. 1 Stunde eine Online-Probenahme erfolgt. Mittels Online-Gaschromatographie wird so die Konzentration der enthaltenen Nitroverbindungen bestimmt. Durch Anpassung der Katalysatorkonzentration in der dem Reaktor zugeführten wässrigen Suspension (s. o.) wird die DNT-Konzentration zwischen 3 und 30 ppm und die ANT-Konzentration zwischen 1 und 200 ppm eingestellt.

Der Reaktor kann unter den genannten Bedingungen über 3 Monate hinweg ohne nennenswerte Unterbrechungen betrieben werden. In dieser Zeit ist keine nennenswerte Katalysatordesaktivierung zu beobachten.

**Beispiel 3**

In einem 5-Liter-Pilotreaktor wurde Dinitrotoluol an einem Pt-Ni/C Katalysator bei einem Druck von 20 bis 25 bar und einer Temperatur von 125 bis 135°C hydriert. Die Konzentrationen von Dinitrotoluol und Aminonitrotoluol in dem Reaktionsgemisch wurden durch Online-GC-Messungen bestimmt.

Das Reaktionsgemisch wurde durch eine Katalysatorabtrenneinheit (Membranfilter) geleitet und der enthaltene Katalysator vom Produkt abgetrennt. Der Katalysator-freie Produktstrom wurde durch ein Dosierventil geleitet. Das Dosierventil war mit einem Gaschromatographen verbunden (Hersteller: Agilent Technologies, Typ 6890). Mit diesem Labor-GC mit Online-Aufbau wurde stündlich eine Probe analysiert.

Der Versuch wurde mit geringer Katalysatorbelastung (0,5 kg/h DNT) gestartet, welche nach einer gewissen Zeit erhöht wurde. Figur 9 zeigt mittels Online-GC gemessene ANT-Konzentrationen (weiße Symbole) und mittels Offline-GC ermittelte ANT-Konzentrationen (schwarze Symbole) als Funktion der Versuchszeit in Tagen. Bei hohen DNT-Strömen wird der Katalysator vergiftet (desaktiviert) und die Konzentration an ANT steigt. Nach Zugabe von frischem Katalysator sinkt die ANT-Konzentration wieder. Die mittels Online- und Offline-GC ermittelten Werte korrelieren.

Durch Einsatz der Online-Messung konnte rechtzeitig Frischkatalysator dosiert werden. Die Offline-Ergebnisse lagen erst mit einer Zeitverzögerung durch die Probenahme und den Probentransport (vom Pilotreaktor zum Labor) vor. Mithilfe der Online-Messung wurde der personelle Aufwand gesenkt.

**Gaschromatographie**

Für die Analytik wurden die nachstehenden Methoden eingesetzt.

### Labormethode (Methode 1):

| | |
|---|---|
| Instrument: | GC 5890 (Agilent Technologies) mit Probensampler |
| Detektor: | Wärmeleitfähigkeitsdetektor |
| Trennsäule: | Restek Rtx 5 Amine, Länge 30 m, Innendurchmesser 0,32 mm, Filmdicke 1 $\mu$m. |
| Trägergas: | Helium |
| Trägergasdruck: | 90 kPa |
| Split: | 1 : 40 |

Temperatur (Injektor): 200 °C - 20 °C Schritte - 300 °C 220°C

Säulenofen: Start Temp.: 125 °C - 5 °C/min bis 285 °C - 40 min

Injektionsvolumen: 0,6 $\mu$l

Probe wird vor der Dosierung mit Anilin verdünnt (Verhältnis 1 : 4)

### Automatisierte Labormethode (Methode 2):

Labor-Gaschromatograph:

| | |
|---|---|
| Instrument: | GC 6890 (Agilent Technologies) modifiziert mit einem automatisierten Flüssigdosierventil (Siemens AG) |
| Detektor: | Flammenionisationsdetektor |
| Trennsäule: | Rtx5 Amine, Länge 30 m, Innendurchmesser 0,32 mm, Filmdicke 1,5 $\mu$m |
| Trägergas: | Helium |
| Trägergasdruck: | 90kPa, constant pressure |
| Dosiermenge: | 0,5 $\mu$l, Splitströmung 70 ml/min |
| Injektortemperatur: | 200 °C |
| Detektortemperatur: | 300 °C |

Ofentemperaturprogramm: Start 125 °C (1 min), 5 °C/min, 285 °C (30 min)

### Automatisierte Labormethode (Methode 3a & 3b & 3c)

Labor-Gaschromatograph:

| | |
|---|---|
| Instrument: | GC 6890 (Agilent Technologies) modifiziert mit einem automatisierten Flüssigdosierventil (Siemens AG) |
| Detektor: | Flammenionisationsdetektor |
| Trennsäule: | Rtx5 Amine, Länge 30 m, Innendurchmesser 0,53 mm, Filmdicke 3 $\mu$m |
| Trägergas: | Helium |
| Injektortemperatur: | 240°C |
| Detektortemperatur: | 300°C |
| Split 1: | 20 (80 ml/min) |
| Injektionsvolumen: | 0,6 $\mu$l |

**Methode 3a:**

Helium 4 ml/min 30 kPa konstanter Fluss
Temperaturrampe: 130 °C @ 1 min -> 285 °C mit 5 °C/min

**Methode 3b:**

Helium 4 ml/min 36 kPa konstanter Fluss
isotherm: 30 min @ 180 °C/Ausheizen 30 min @ 250 °C

**Methode 3c:**

Helium 4 ml/min 30 kPa konstanter Fluss
Temperaturrampe: 170 °C @ 30 min -> 280 °C mit 20 °C/min

**Patentansprüche**

1. Verfahren zur Herstellung von aromatischen Aminen durch Hydrierung von Nitroaromaten in Gegenwart von Katalysatoren, wobei in einem Reaktor ein fluides, Amine enthaltendes Reaktionsgemisch entsteht, bei dem ein Gaschromatogramm des Reaktionsgemisches zur Konzentrationsbestimmung von Nitro- und Nitrosoverbindungen in dem Reaktionsgemisch erstellt und ausgewertet wird, wobei das Ergebnis der Konzentrationsbestimmung der Nitro- und Nitrosoverbindungen zur, vorzugsweise automatisierten, Prozess-Steuerung oder Regelung der Hydrierung verwendet wird, **dadurch gekennzeichnet, dass** Proben automatisiert über einen Bypass dem Gaschromatographen online zugeführt werden.

2. Verfahren nach Anspruch 1, **dadurch gekennzeichnet, dass** die Konzentration an Nitro- und Nitrosoverbindungen im Bereich von 1 bis 200 ppm eingestellt werden.

3. Verfahren nach einem der Ansprüche 1 oder 2, **dadurch gekennzeichnet, dass** die Prozess-Steuerung oder Regelung der Hydrierung eine Änderung der Zudosierung von Katalysator und/oder Nitroaromaten beinhaltet.

4. Verfahren nach Anspruch 1, **dadurch gekennzeichnet, dass** der Bypass mit einem automatisierten Flüssigdosierventil zur Probenahme, gegebenenfalls mit vorgeschaltetem Partikelfilter, ausgerüstet ist.

5. Verfahren nach einem der Ansprüche 1 bis 4, **dadurch gekennzeichnet, dass** die aromatischen Amine Aniline, Toluidine, Naphthyldiamine, Xylylendiamine oder Toluylendiamine sind.

6. Verfahren nach einem der Ansprüche 1 bis 5, **dadurch gekennzeichnet, dass** Toluylendiamin durch Hydrierung von Dinitrotoluol hergestellt wird und von in dem Reaktionsgemisch enthaltenem Dinitrotoluol und Aminonitrotoluol eine Flächenbestimmung der entsprechenden Peaks im Chromatogramm zur Konzentrationsbestimmung durchgeführt wird.

7. Verfahren nach Anspruch 6, **dadurch gekennzeichnet, dass** die Dinitrotoluol-Konzentration im flüssigen Produktaustrag aus dem Reaktor auf einen Wert im Bereich von 1 bis 100 Gew.-ppm, bezogen auf das Gesamtgewicht des flüssigen Produktaustrages aus dem Reaktor, eingestellt wird.

8. Verfahren nach einem der Ansprüche 1 bis 7, **dadurch gekennzeichnet, dass** die Hydrierung in Gegenwart von Nickel enthaltenden Katalysatoren durchgeführt wird.

9. Verfahren nach einem der Ansprüche 1 bis 8, **dadurch gekennzeichnet, dass** die Dosierung des Reaktionsgemisches in den Gaschromatographen mittels eines auf mindestens 80 °C beheizten automatisierten Flüssigdosierventils erfolgt.

10. Verfahren nach einem der Ansprüche 1 bis 9, **dadurch gekennzeichnet, dass** die Probe des Reaktionsgemisches bei der Probennahme bis zur Analyse bei einer Temperatur von mindestens 80 °C gehalten wird oder nach der Probennahme mit einem Lösungsmittel verdünnt wird.

11. Verfahren nach einem der Ansprüche 1 bis 10, **dadurch gekennzeichnet, dass** die Probenahme für die Konzentrationsbestimmung in einem Bypass des flüssigen Produktaustrags hinter einer Produktabtrenneinheit oder in einem Bypass des flüssigen Produktaustrags zwischen Reaktor und Produktabtrenneinheit erfolgt.

12. Verfahren zur Regelung der Konzentration von Nitro- und Nitrosoverbindungen in einem Verfahren zur Herstellung von aromatischen Aminen durch Hydrierung von Nitroaromaten in Gegenwart von Katalysatoren nach einem der Ansprüche 1 bis 11, wobei in einem Reaktor ein fluides, Amine enthaltendes Reaktionsgemisch entsteht, umfassend die folgenden Schritte:

a) Aufnahme eines Gaschromatogramms des Reaktionsgemisches, wobei Proben automatisiert über einen Bypass dem Gaschromatographen online zugeführt werden,

b) Eingabe des in Schritt a) aufgenommenen Gaschromatogramms in eine Gaschromatogramm-Auswerteeinheit (Auswertesoftware) und/oder in ein chemometrisches Kalibrationsmodell zur Bestimmung der Ist-Konzentration der Nitro- und Nitrosoverbindungen im Reaktionsgemisch,

c) Bestimmung einer Soll-Ist-Abweichung der Konzentration der Nitro- und Nitrosoverbindungen im Reaktionsgemisch,

d) Regelung eines oder mehrerer Prozessparameter der Hydrierung, basierend auf der in Schritt c) bestimmten Soll-Ist-Abweichung.

13. Verfahren nach Anspruch 12, **dadurch gekennzeichnet, dass** in Schritt d) die Katalysatorzudosierung und/oder die Zudosierung von Nitroaromaten verändert bzw. geregelt wird.

14. Vorrichtung zur Durchführung des Verfahrens zur Herstellung von aromatischen Aminen nach einem der Ansprüche 1 bis 11 mit einem Reaktor zur Hydrierung von Nitroaromaten in Gegenwart von Katalysatoren, wobei ein Reaktionsgemisch entsteht, mit einem Gaschromatographen zur Erstellung eines Gaschromatogramms zur Konzentrationsbestimmung von Nitro- und Nitrosoverbindungen in dem Reaktionsgemisch, wobei die Vorrichtung eingerichtet ist, um die Aufnahme von Gaschromatogrammen von Proben, die automatisiert über einen Bypass dem Gaschromatographen online zugeführt werden, vorzunehmen, mit einer Auswerteeinheit zur Konzentrationsbestimmung von Nitroaromaten, sowie einer Regelungseinheit zur Regelung der Katalysatorzudosierung und/oder Zudosierung von Nitroaromaten.

15. Vorrichtung nach Anspruch 14, **dadurch gekennzeichnet, dass** sie umfasst:

a) Mittel (202, 204) zur Aufnahme eines Gaschromatogramms des Reaktionsgemisches, von Proben, die automatisiert über einen Bypass dem Gaschromatographen online zugeführt werden,

b) eine Gaschromatogramm-Auswerteeinheit (Auswertesoftware) (206) und/oder ein chemometrisches Kalibrationsmodell (210) zur Auswertung des Gaschromatogramms zur Bestimmung einer Ist-Konzentration an Nitro- und Nitrosoverbindungen im Reaktionsgemisch aus dem Chromatogramm,

c) Mittel (212) zur Bestimmung einer Soll-Ist-Abweichung,

d) Mittel (212) zur Regelung eines oder mehrerer Prozessparameter der Hydrierung, insbesondere der Dosierung der aromatischen Nitroverbindung (214), und/oder Dosierung des Katalysators (216) basierend auf der Soll-Ist-Abweichung.

## Claims

1. A process for preparing aromatic amines by hydrogenation of nitroaromatics in the presence of catalysts, forming a fluid, amine-comprising reaction mixture in a reactor, in which process a gas chromatogram of the reaction mixture is produced and evaluated to determine the concentration of nitro and nitroso compounds in the reaction mixture, with the result of the determination of the concentration of nitro and nitroso compounds being used for preferably automated process control or regulation of the hydrogenation, wherein samples are fed on-line automatically via a bypass to the gas chromatograph.

2. The process according to claim 1, wherein the concentration of nitro and nitroso compounds is set in the range from 1 to 200 ppm.

3. The process according to either of claims 1 and 2, wherein the process control or regulation of the hydrogenation comprises alteration of the introduction of catalyst and/or nitroaromatics.

4. The process according to claim 1, wherein the bypass is equipped with an automated liquid metering valve for sampling, optionally with upstream particle filter.

5. The process according to any of claims 1 to 4, wherein the aromatic amines are anilines, toluidines, naphthyldiamines, xylylenediamines or toluenediamines.

6. The process according to any of claims 1 to 5, wherein toluenediamine is prepared by hydrogenation of dinitrotoluene and the area of the peaks of dinitrotoluene and aminonitrotoluene in the chromatogram of the reaction mixture is determined to determine the concentration.

7. The process according to claim 6, wherein the dinitrotoluene concentration in the liquid product output from the reactor is set to a value in the range from 1 to 100 ppm by weight, based on the total weight of the liquid product output from the reactor.

8. The process according to any of claims 1 to 7, wherein the hydrogenation is carried out in the presence of nickel-comprising catalysts.

9. The process according to any of claims 1 to 8, wherein the introduction of the reaction mixture into the gas chromatograph is carried out by means of an automated liquid metering valve heated to at least 80°C.

10. The process according to any of claims 1 to 9, wherein the sample of the reaction mixture is maintained at a temperature of at least 80°C from sampling to analysis or is diluted with a solvent after sampling.

11. The process according to any of claims 1 to 10, wherein sampling for the concentration determination is carried out in a bypass for the liquid product output downstream of a product isolation unit or in a bypass for the liquid product output between reactor and product isolation unit.

12. A method of regulating the concentration of nitro and nitroso compounds in a process for preparing aromatic amines by hydrogenation of nitroaromatics in the presence of catalysts according to any of claims 1 to 11, forming a fluid, amine-comprising reaction mixture in a reactor, which comprises the following steps:

   a) recording of a gas chromatogram of the reaction mixture, wherein samples are fed online automatically via a bypass to the gas chromatograph,
   b) input of the gas chromatogram recorded in step a) into a gas chromatogram evaluation unit (evaluation software) and/or into a chemometric calibration model in order to determine the actual concentration of the nitro and nitroso compounds in the reaction mixture,
   c) determination of an intended-actual deviation of the concentration of nitro and nitroso compounds in the reaction mixture,
   d) regulation of one or more process parameters of the hydrogenation, on the basis of the intended/actual deviation determined in step c).

13. The method according to claim 12, wherein in step d) the introduction of catalyst and/or the introduction of nitroaromatics is altered or regulated.

14. An apparatus for carrying out the process for preparing aromatic amines according to any of claims 1 to 11, which comprises a reactor for the hydrogenation of nitroaromatics in the presence of catalysts, forming a reaction mixture, with a gas chromatograph for producing a gas chromatogram to determine the concentration of nitro and nitroso compounds in the reaction mixture, wherein the apparatus is equipped to record gas chromatograms of samples which are fed online automatically via a bypass to the gas chromatograph, with an evaluation unit for determining the concentration of nitroaromatics and a regulator unit for regulating the introduction of catalyst and/or introduction of nitroaromatics.

15. The apparatus according to claim 14, which comprises:

   a) means (202, 204) for recording a gas chromatogram of the reaction mixture of samples which are fed online automatically via a bypass to the gas chromatograph,
   b) a gas chromatogram evaluation unit (evaluation software) (206) and/or a chemometric calibration model (210) for evaluating the gas chromatogram to determine an actual concentration of nitro and nitroso compounds in the reaction mixture from the chromatogram,
   c) means (212) for determining an intended-actual deviation,
   d) means (212) for regulating one or more process parameters of the hydrogenation, in particular the introduction of the aromatic nitro compound (214) and/or introduction of the catalyst (216) on the basis of the intended/actual deviation.

**Revendications**

1. Procédé de fabrication d'amines aromatiques par hydrogénation de composés nitro aromatiques en présence de catalyseurs, selon lequel un mélange réactionnel fluide contenant des amines est formé dans un réacteur, un chromatogramme gazeux du mélange réactionnel étant établi et exploité pour déterminer la concentration en composés nitro et nitroso dans le mélange réactionnel, le résultat de la détermination de la concentration en composés nitro et nitroso étant utilisée pour la commande de processus ou la régulation, de préférence automatisée, de l'hydrogénation, **caractérisé en ce que** des échantillons sont introduits en ligne, de manière automatisée, par le biais d'une dérivation, dans le chromatographe gazeux.

2. Procédé selon la revendication 1, **caractérisé en ce que** la concentration en composés nitro et nitroso est ajustée dans la plage allant de 1 à 200 ppm.

3. Procédé selon l'une quelconque des revendications 1 ou 2, **caractérisé en ce que** la commande de processus ou la régulation de l'hydrogénation comprend une modification du dosage du catalyseur et/ou des composés nitro aromatiques.

4. Procédé selon la revendication 1, **caractérisé en ce que** la dérivation est munie d'une vanne de dosage de liquide automatisée pour l'échantillonnage, éventuellement avec un filtre à particules en amont.

5. Procédé selon l'une quelconque des revendications 1 à 4, **caractérisé en ce que** les amines aromatiques sont des anilines, des toluidines, des naphtyldiamines, des xylylène-diamines ou des toluylène-diamines.

6. Procédé selon l'une quelconque des revendications 1 à 5, **caractérisé en ce que** de la toluylène-diamine est fabriquée par hydrogénation de dinitrotoluène et une détermination des surfaces des pics correspondants du dinitrotoluène et de l'aminonitrotoluène contenus dans le mélange réactionnel sur le chromatogramme est réalisée pour déterminer la concentration.

7. Procédé selon la revendication 6, **caractérisé en ce que** la concentration en dinitrotoluène dans la sortie de produits liquide du réacteur est ajustée à une valeur dans la plage allant de 1 à 100 ppm en poids, par rapport au poids total de la sortie de produits liquide du réacteur.

8. Procédé selon l'une quelconque des revendications 1 à 7, **caractérisé en ce que** l'hydrogénation est réalisée en présence de catalyseurs contenant du nickel.

9. Procédé selon l'une quelconque des revendications 1 à 8, **caractérisé en ce que** le dosage du mélange réactionnel dans le chromatographe gazeux a lieu au moyen d'une vanne de dosage de liquide automatisée portée à au moins 80 °C.

10. Procédé selon l'une quelconque des revendications 1 à 9, **caractérisé en ce que** l'échantillon du mélange réactionnel lors de l'échantillonnage jusqu'à l'analyse est maintenu à une température d'au moins 80 °C ou dilué avec un solvant après l'échantillonnage.

11. Procédé selon l'une quelconque des revendications 1 à 10, **caractérisé en ce que** l'échantillonnage pour la détermination de la concentration a lieu dans une dérivation de la sortie de produits liquide après une unité de séparation de produits ou dans une dérivation de la sortie de produits liquide entre le réacteur et l'unité de séparation de produits.

12. Procédé de régulation de la concentration en composés nitro et nitroso dans un procédé de fabrication d'amines aromatiques par hydrogénation de composés nitro aromatiques en présence de catalyseurs selon l'une quelconque des revendications 1 à 11, selon lequel un mélange réactionnel fluide contenant des amines est formé dans un réacteur, comprenant les étapes suivantes :

   a) l'enregistrement d'un chromatogramme gazeux du mélange réactionnel, des échantillons étant introduits en ligne, de manière automatisée, par le biais d'une dérivation, dans le chromatographe gazeux,
   b) l'entrée du chromatogramme gazeux enregistré à l'étape a) dans une unité d'exploitation de chromatogrammes gazeux (logiciel d'exploitation) et/ou dans un modèle de calibration chimiométrique pour déterminer la concentration réelle en composés nitro et nitroso dans le mélange réactionnel,

c) la détermination d'une déviation entre la valeur cible et réelle de la concentration en composés nitro et nitroso dans le mélange réactionnel,

d) la régulation d'un ou de plusieurs paramètres de processus de l'hydrogénation à partir de la déviation entre la valeur cible et réelle déterminée à l'étape c).

**13.** Procédé selon la revendication 12, **caractérisé en ce qu'**à l'étape d), le dosage du catalyseur et/ou le dosage des composés nitro aromatiques sont modifiés ou régulés.

**14.** Dispositif pour la réalisation du procédé de fabrication d'amines aromatiques selon l'une quelconque des revendications 1 à 11, comprenant un réacteur pour l'hydrogénation de composés nitro aromatiques en présence de catalyseurs, dans lequel un mélange réactionnel se forme, un chromatographe gazeux pour l'établissement d'un chromatogramme gazeux pour déterminer la concentration en composés nitro et nitroso dans le mélange réactionnel, le dispositif étant conçu pour réaliser l'enregistrement de chrommatogrammes gazeux d'échantillons qui sont introduits en ligne, de manière automatisée, par le biais d'une dérivation, dans le chromatographe gazeux, une unité d'exploitation pour déterminer la concentration en composés nitro aromatiques, ainsi qu'une unité de régulation pour la régulation du dosage du catalyseur et/ou du dosage des composés nitro aromatiques.

**15.** Dispositif selon la revendication 14, **caractérisé en ce qu'**il comprend :

a) des moyens (202, 204) pour l'enregistrement d'un chromatogramme gazeux du mélange réactionnel, par des échantillons qui sont introduits en ligne, de manière automatisée, par le biais d'une dérivation, dans le chromatographe gazeux,

b) une unité d'exploitation de chromatogrammes gazeux (logiciel d'exploitation) (206) et/ou un modèle de calibration chimiométrique (210) pour l'exploitation du chromatogramme gazeux pour la détermination d'une concentration réelle en composés nitro et nitroso dans le mélange réactionnel à partir du chromatogramme,

c) un moyen (212) pour déterminer une déviation entre la valeur cible et réelle,

d) un moyen (212) pour réguler un ou plusieurs paramètres de processus de l'hydrogénation, notamment le dosage du composé nitro aromatique (214) et/ou le dosage du catalyseur (216) à partir de la déviation entre la valeur cible et réelle.

```
┌─────────────────────────────────────────────────┐
│                      100                        │
│           Dosierung von Nitroaromaten            │
└─────────────────────────────────────────────────┘
                        │
                        ▼
┌─────────────────────────────────────────────────┐
│                      102                        │
│  Reaktionsgemisch $G_R$ mit Soll-Konzentration an Nitroaromaten │
└─────────────────────────────────────────────────┘
                        │
                        ▼
┌─────────────────────────────────────────────────┐
│                      104                        │
│                    Bypass                        │
└─────────────────────────────────────────────────┘
                        │
                        ▼
┌─────────────────────────────────────────────────┐
│                      106                        │
│                     i -1                         │
└─────────────────────────────────────────────────┘
                        │
                        ▼
┌─────────────────────────────────────────────────┐
│                      108                        │
│           Gaschromatogramm Gemisch $G_R$         │
└─────────────────────────────────────────────────┘
                        │
                        ▼
┌─────────────────────────────────────────────────┐
│                      110                        │
│   Ist-Konzentration an Nitroaromaten im Gemisch $G_R$ │
└─────────────────────────────────────────────────┘
                        │
                        ▼
┌─────────────────────────────────────────┐   ┌──────────────┐
│                 112                      │──▶│     114      │
│            Soll-Ist Differenz            │   │   Regelung   │
└─────────────────────────────────────────┘   └──────────────┘
                        │
                        ▼
┌─────────────────────────────────────────────────┐
│                      116                        │
│                     i +1                         │
└─────────────────────────────────────────────────┘
```

**Fig. 1**

Fig. 2

**Fig. 3**

**Fig. 4**

**Fig. 5**

**Fig. 6**

**Fig. 7**

**Fig. 8**

Fig. 9

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2044657 A
- EP 1138665 B1
- US 5856577 A
- WO 03066571 A1
- WO 2006089906 A
- DE 3245318 A1
- DE 3040631 A1
- WO 03066571 A
- EP 1445246 A
- US 5121337 A
- EP 0094876 B1
- EP 1445246 A2
- EP 1138665 A
- WO 2005037768 A
- WO 0035852 A
- DE 19857409 A
- EP 0124010 A
- EP 2009067610 W
- EP 10162924 A
- EP 21310302 A
- WO 03068724 A
- EP 1161297 A1
- EP 1165231 A1
- WO 2008145179 A1
- WO 2008138784 A1
- WO 200035852 A1
- EP 1161297 A